# EUROPEAN PATENT SPECIFICATION

(11) **EP 2 917 244 B1**
(45) Date of publication and mention of the grant of the patent: **06.01.2021**
(21) Application number: 13853308.8
(22) Date of filing: 12.11.2013
(51) Int. Cl.: C07K 19/00, A61K 47/68, A61P 35/00, A61P 35/04, C07K 14/81, C07K 16/28

(54) **APROTININ-DERIVED POLYPEPTIDE-ANTIBODY CONJUGATES**
AUS APROTININ GEWONNENE ANTIKÖRPERKONJUGATE
CONJUGUÉS POLYPEPTIDE DÉRIVÉ D'APROTININE-ANTICORPS

(30) Priority: 12.11.2012 US 201261725365 P
(43) Date of publication of application: 16.09.2015
(73) Proprietor: Angiochem Inc., Montréal, QC H2X 3Y7 (CA)
(72) Inventor: TRIPATHY, Sasmita, Pierrefonds Québec H9H 2G2 (CA); DEMEULE, Michel, Beaconsfield Québec H9W 1Z2 (CA); CURRIE, Jean-Christophe, Repentigny Québec J6A 6C9 (CA)
(74) Representative: Lahrtz, Fritz
(86) International application number: PCT/CA2013/050863
(87) International publication number: WO 2014/071531

(56) References cited:
- WO-A1-2007/009229
- WO-A1-2008/144919
- WO-A1-2010/121379
- WO-A1-2012/000118
- WO-A2-2011/060206
- US-A1- 2009 016 959
- US-A1- 2013 280 281
- ]!AN E LACHOWICZ ET AL: "Design of new Angiopep-2-anti-EGFR and Angiopep-2-anti-Her2 derivatives with increased blood-brain barrier permeability for treatment of brain tumors", NEURO-ONCOLOGY : OFFICIAL JOURNAL OF THE WORLD FEDERATION OF NEURO-ONCOLOGY, OXFORD : OXFORD UNIV. PRESS, GB, vol. 14, no. Suplement, 15 January 2012 (2012-01-15), pages VI30-VI31, XP008178859, ISSN: 1523-5866
- LACHOWICZ, J.E. ET AL.: 'Design of new Angiopep-2-anti-EGFR and Angiopep-2-anti-Her2 derivatives with increased blood-brain barrier permeability for treatment of brain tumors' NEURO-ONCOLCOLOGY. vol. 14, no. SUPPL, 15 October 2012, WASHINGTON, D.C., pages VI25 - VI37, XP008178859
- LACHOWICZ, J.E. ET AL.: 'ANG4043: A new brain-penetrant peptide-antibody conjugate that reduces tumor growth in a HER2-positive orthotopic tumor model. [abstract].' PROCEEDINGS OF THE 104TH ANNUAL MEETING OF THE AMERICAN ASSOCIATION FOR CANCER RESEARCH vol. 73, no. 8, 15 April 2013, WASHINGTON, DC. PHILADELPHIA (PA), XP008178860

## Description

### CROSS-REFERENCE TO RELATED APPLICATIONS

This application claims the benefit of the priority date of U.S. Provisional Application No. 61/725,365, which was filed November 12, 2012.

### FIELD OF THE INVENTION

The present invention relates to protein conjugates in which one or more Kunitz-type protease inhibitors *(e.g.,* aprotinin) or derivatives thereof *(e.g.,* an aprotinin-derived polypeptide) are conjugated to an antibody moiety in the manner described herein; methods by which the conjugates are synthesized for pharmaceutical use or diagnostic use (*e.g.,* use as imaging agents); physiologically acceptable compositions including them; and methods of administering the conjugates to patients for the diagnosis and treatment of, for example, cancer and neurological disorders.

### BACKGROUND OF THE INVENTION

While the blood-brain barrier (BBB) protects the central nervous system (CNS) from harmful substances (*e.g.,* accidentally ingested toxins), it also prevents therapeutic proteins from accessing the brain and spinal cord and, therefore, presents a major obstacle in treating disorders of the CNS. As a general rule, only lipophilic molecules smaller than about 500 Daltons traverse the BBB. Many drugs that show promising results in animal studies of CNS disease are considerably larger than that, and protein therapeutics are generally excluded from the CNS altogether due to the negligible permeability of the brain capillary endothelial wall to drugs of that size and complexity.

The strategies currently being pursued to enhance delivery of protein therapeutics to the CNS can be divided into three categories. The first category includes invasive procedures such as direct intraventricular administration of drugs and intra-carotid infusion of hyperosmolar solutions that temporarily disrupt the BBB. The second category includes pharmacologically-based strategies that are aimed at increasing the lipid solubility of proteins through the BBB. In the third category are delivery regimes in which the therapeutic agent is attached to a protein that acts as a vector or receptor-targeted delivery vehicle with respect to the BBB. This approach is advantageous in that it is highly specific and efficacious, results in minimal untoward effects, and is broadly applicable. The document WO2008144919 discloses antibody-peptide conjugates, using different linkers to those of the present application. The document WO2012000118 also discloses a number of linkers that can be used to link antibodies with other agents such as peptides.

### SUMMARY OF THE INVENTION

The scope of the invention is defined in the claims. Any discussion of embodiments not falling within the scope of the claims is for reference only.

In a first aspect, the present invention features protein conjugates that include an antibody moiety and one or more Kunitz-type protease inhibitors *(e.g.,* aprotinin) or derivatives thereof (*e.g.,* an aprotinin-derived polypeptide). Both the antibody moiety and the polypeptide that transports the conjugate across the BBB are discussed at length below. The protein conjugates are further defined by the inclusion of a linker; They can be further defined by the number of polypeptides conjugated to each antibody moiety; and/or by the positions at which the antibody moiety and the polypeptides are conjugated. Moreover, within a given conjugate, where more than one polypeptide is included, each polypeptide is linked to only the antibody moiety. In other words, no polypeptide is linked, via a linker or otherwise, to another polypeptide within the conjugate.

In one embodiment, the protein conjugates include an antibody moiety and a polypeptide that includes the amino acid sequence Thr₁-Phe₂-Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:117) or a biologically active analog or fragment of SEQ ID NO:117. As indicted by "Cys₇/Ser₇", the amino acid residue at position 7 can be cysteine or serine. In protein conjugates that include this polypeptide (SEQ ID NO:117) or any other polypeptide described herein, the antibody moiety can be conjugated to the polypeptide at one or more of the following positions on the polypeptide: the N-terminal amino acid residue (e.g., Thr₁ where the polypeptide consists of SEQ ID NO:117); the C-terminal amino acid residue (*e.g.,* Tyr₁₉ where the polypeptide consists of SEQ ID NO:117); or a lysine residue between the N-terminus and the C-terminus (*e.g.,* Lys₁₀ or Lys₁₅ in SEQ ID NO:117).

We use the term "protein conjugate" to refer to an amino acid-based compound formed of molecularly coupled parts. In the present invention, the parts include an antibody moiety and the polypeptide(s) coupled thereto (*e.g.,* via a linker). An "amino acid-based compound" is one that includes primarily, but not necessarily exclusively, amino acid residues (*i.e*., one that includes only amino acid residues (as in a conventional fusion protein) or one that includes amino acid residues and other components). As noted above, the protein conjugates can include a linker, which may be a chemical compound. Such conjugates would include primarily, but not exclusively, amino acid residues, as the antibody moiety and polypeptide are made of amino acids and the linker is a chemical compound. The protein conjugates may also include a detection agent (*e.g.,* a fluorophor, radioisotope, dye, or the like). The detectable agent may not include amino acid residues, and in that event, we may also refer to a protein conjugate containing such detectable agents as an amino acid-based compound. The invention encompasses methods of using detectable conjugates in diagnostic methods, including diagnostic imaging.

For the sake of added clarity, we note that the antibody moiety and the polypeptide(s) are distinct from one another *(i.e.,* they are not identical to one another). However, any protein conjugate can include more than one polypeptide, and the two, three, four, or more polypeptides may be the same (*i.e*., may have an identical amino acid sequence) or may differ from one another (*i.e*., the polypeptides conjugated to the antibody moiety may have different amino acid sequences). With respect to the compositions and methods of the invention, we use the term "include(s)" to mean "comprising." For example, in the embodiment described above, the protein conjugates comprise an antibody moiety and a polypeptide that, in turn, comprises the amino acid sequence represented by SEQ ID NO:117. In any instance, unless the context specifically indicates otherwise, the compositions of the invention, their component parts *(e.g.,* the antibody moiety and the polypeptide), and the methods of the invention can either comprise or consist of the recited elements or steps. For example, a given polypeptide can comprise or consist of the recited sequence (*e.g.,* SEQ ID NO:117).

As indicated above, the protein conjugates of the present invention can include a polypeptide as described herein or a biologically active analog or fragment thereof. We use the term "biologically active" with respect to the polypeptide portion of the protein conjugates to mean that the analog or fragment of a referenced polypeptide retains sufficient activity in a physiological setting to be useful. Since the polypeptide portion of the protein conjugates is responsible for transporting the conjugate and/or the antibody moiety to which the polypeptides are attached across the BBB, an analog or fragment of a referenced polypeptide is biologically active when it has the ability to transport the conjugate and/or the antibody moiety to which it is attached across the BBB. This property can be tested *in vivo* or in *ex vivo* models, and biologically active analogs and fragments of a referenced polypeptide may be more or less effective in this transport than the referenced polypeptide. In particular, the efficacy of a fragment or analog may be lower than that of the referenced polypeptide as long as it remains high enough to achieve a desired outcome (*e.g.,* as long as the conjugate in which it is included achieves a clinically beneficial result).

A "fragment" of a referenced polypeptide is a continuous or contiguous portion of the referenced polypeptide (*e.g.,* a fragment of a polypeptide that is ten amino acids long can be any 2-9 contiguous residues within that polypeptide). An "analog" of a referenced polypeptide is any polypeptide having a sequence that is similar to, but not identical to, the sequence of the referenced polypeptide or a portion thereof. Thus, a polypeptide that includes one or more amino acid substitutions, additions, or deletions of an amino acid residue (or any combination thereof) is an analog of the referenced sequence. Fragments and analogs of polypeptides suitable for inclusion in the present protein conjugates are further described below.

As indicated, any of the protein conjugates of the invention include a linker between the antibody moiety and the polypeptide(s) of the conjugate. The conjugates can be assembled by methods known in the art as "click chemistry." Perhaps the most prominent example of a click reaction is the copper-catalyzed azide-alkyne cycloaddition reaction (CuAAC), and such reactions can be employed to generate the conjugates of the present invention. These reactions are useful *in vivo* because neither azide nor terminal alkyne functional groups are generally present in natural systems. Despite the attractive features of CuAAC-based click chemistry, copper may hinder bioorthogonal conjugation in living cells or whole organisms, and the present methods can also be carried out with copper-free click chemistry *(i.e.,* the present conjugates can be assembled with copper-free click chemistry).

In the present invention, the linker is a chemical compound that includes a group reactive with a primary amine. Thus, the linker can be a monofluorinated cyclooctyne (MFCO), bicyclo[6.1.0]nonyne (BCN), or dibenzocyclooctyne (DBCO). Suitable linkers and methods of incorporating them into the present conjugates are further described below.

In the polypeptide represented by SEQ ID NO:117, the residue at numbered position 7 can be a cysteine residue or a serine residue, and this substitution can be made in any of polypeptides described herein. Thus, useful polypeptides can include the amino acid sequence Thr₁-Phe₂-Phe₃-Tyr₄-Gly₅-Gly₆-Cys₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:67), the amino acid sequence Thr₁-Phe₂-Phe₃-Tyr₄-Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:97), or a biologically active fragment or analog of the polypeptide represented by SEQ ID NO:67 or SEQ ID NO:97. For ease of reading, we will not repeat the phrase "or a biologically active fragment or analog thereof' at every possible occasion. It is to be understood that where a referenced polypeptide is useful, a biologically active fragment or analog thereof is also useful.

Where the protein conjugate includes an analog of SEQ ID NO:117, the analog can comprise at least 13 amino acid residues (*e.g.,* 13-19 amino acid residues, inclusive) that are invariant relative to SEQ ID NO:117. As an analog may include additional amino acid residues, the analog may be longer than the 19 amino acid residues of SEQ ID NO:117. Regardless of length or the exact nature of the difference(s), the degree of similarity can be expressed as the "percent identity" between the sequence of the analog and the sequence of the referenced polypeptide (here, SEQ ID NO:117). Analogs characterized in this manner are further described below. In any instance where the polypeptide is an analog of SEQ ID NO:117, the lysine residue at numbered position 10 and/or the lysine residue at numbered position 15 can remain invariant. That is, analogs can include a lysine residue at position 10 and/or at position 15. One of ordinary skill in the art would recognize that the absolute position of a given residue may vary in some cases. For example, the lysine residues just mentioned will be present at positions equivalent to positions 10 and/or 15 should the analog include more than or less than the first nine amino acid residues of SEQ ID NO:117. For example, in an analog that includes one additional amino acid residue at the N-terminus, the lysine residues at positions 10 and 15 of the reference sequence would appear at positions 11 and 16 of the analog.

In other analogs of SEQ ID NO:117, Asn₁₂ can be substituted with Gln, Asn₁₃ can substituted with Gln, and/or Phe₁₄ can be substituted with Tyr or Trp. In other useful analogs of SEQ ID NO:117, a cysteine residue is added to the amino- and/or carboxy-terminal end of the polypeptide. A cysteine residue can be added at a terminal position whether that position is the threonine residue at position 1 of SEQ ID NO:117, the tyrosine residue at position 19 of SEQ ID NO:117, or a residue internal to either of those that was generated by a deletion of one or more residues from the amino- and/or carboxy-terminal end of the polypeptide. In other words, an N- and/or C-terminal cysteine residue can be added to a full-length polypeptide or to a fragment or analog thereof. Useful analogs can include the sequence: Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:118); Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:119); Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:120); Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:121); or Lys₁₀-Arg₁₁-Asn₁₂-Asn₁₃-Phe₁₄-Lys₁₅-Tyr₁₉₋Cys (SEQ ID NO:122). We have numbered the amino acid residues as we have in order to make it more apparent how the analog relates to SEQ ID NO:117. For example, Phe₃ is the third amino acid residue of SEQ ID NO:117 but, obviously, the first amino acid residue in the analog of SEQ ID NO:117 that is represented by SEQ ID NO:118.

Where the protein conjugate includes a fragment of SEQ ID NO:117, the fragment can include at least six (*e.g.,* 6-18, inclusive) contiguous amino acid residues of SEQ ID NO:117. For example, the fragment can include or consist of the sequence Lys₁₀-Arg₁₁-Asn₁₂-Asn₁₃-Phe₁₄-Lys₁₅ (SEQ ID NO:123).

In any embodiment, the polypeptide can include amino acid residues in the D-form, the L-form, or both forms. For example, one or more (and up to all residues) can be in the L-form or the D-form.

With respect to the configuration of the protein conjugates, the conjugates can include one antibody moiety and, attached thereto, a variable number of polypeptides. The number of polypeptides will be governed by the number of suitable points of attachment on the antibody moiety. As the linkers described herein can react with a primary amine group, the linkers may attach polypeptides to the antibody moiety at one or more of the lysine residues within the antibody moiety. Alternatively, the linker may also react with (and therefore become bound to) a thiol group or a carbohydrate group on the antibody moiety. Linkers may reside at as few as 1-10 lysine residues *(i.e.,* 1, 2, 3, 4, 5, 6, 7, 8, 9, or 10 polypeptides can be conjugated at distinct lysine residues within the antibody moiety). In particular embodiments, about 15 *(e.g.,* 14-16), about 30, about 40, about 50, about 60, about 70, about 80, or about 90 lysine residues may be occupied. The number of polypeptides within a conjugate can also be expressed as a percentage of the number of residues within the antibody moiety available for conjugation. More specifically, about 10% to up to 100% of the lysine residues can be conjugated to a polypeptide.

The particular antibodies falling within the scope of this invention are defined in the claims. In any embodiment, the antibody moiety can be of the IgG class. In any embodiment, the antibody moiety can be a tetrameric antibody, a single chain antibody (scFv), a fragment of a tetrameric antibody (*e.g.,* an Fab fragment, an F(ab')2 fragment) or a biologically active variant of any of these antibody moieties. We use the term "biologically active" with respect to the antibody portion of the protein conjugates to mean that the variant of the antibody retains sufficient activity in a physiological setting to be useful. Since the antibody moiety of the protein conjugate selectively binds a biological target, a variant of a referenced antibody moiety is biologically active when it has the ability to selectively bind that target to a useful extent. A biologically active variant of an antibody moiety may have, for example, a different affinity for the biological target *(e.g.,* a cellular receptor) than the referenced antibody moiety. The affinity may be lower than that of the referenced antibody moiety as long as it remains high enough to achieve a desired outcome (*e.g.,* as long as the conjugate in which it is included achieves a clinically beneficial result).

The antibody moiety can be a human, chimeric or humanized antibody moiety or a biologically active variant thereof. For ease of reading, we will not repeat the phrase "or a biologically active variant thereof' at every occasion. It is to be understood that where an antibody moiety is useful in the context of the present invention, a biologically active variant thereof is also useful. The antibody moiety can be a monoclonal antibody or a polyclonal antibody, a single chain antibody constructed from monoclonal or polyclonal antibodies, or a fragment (*e.g.,* an Fab or F(ab')2 fragment) of a monoclonal or polyclonal antibody.

With respect to targets, the antibody moiety *(e.g.,* a tetrameric antibody, a biologically active variant thereof, an scFv, Fab fragment, or F(ab')2 fragment, or biologically active variants thereof, regardless of class *(i.e.,* whether of the IgG class or another class) and whether human, humanized, chimeric, monoclonal, or having any other attribute or characteristic described herein) can specifically bind a growth factor receptor or a cytokine receptor *(e.g.,* an interleukin receptor). The growth factor receptor can be a receptor bound by a member of the epidermal growth factor (EGF) family. Examples of receptors for proteins in the EGF family include an EGF receptor (EGFR), a heparin-binding EGF-like growth factor receptor (HB-EGFR), an amphiregulin receptor (AR), an epiregulin receptor (EPR), an epigen receptor, a betacellulin receptor, and a receptor for a neuregulin (*e.g.,* a receptor for neuregulin-1, neuregulin-2, neuregulin-3, or neuregulin-4). In particular, the antibody moiety can be trastuzumab, cetuximab, or panitumumab, an scFv comprising the variable regions of the heavy and light chains of trastuzumab, cetuximab, or panitumumab, or a biologically active variant of these tetrameric or single chain antibodies. For example, an Fab or F(ab')2 fragment of trastuzumab, cetuximab, or panitumumab. We have conducted studies with antibodies having a sequence that is about 99% homologous to trastuzumab. With respect to function, the antibody moiety can be a chemotherapeutic agent or an anti-inflammatory agent.

In a second aspect, the invention features pharmaceutical compositions that include a protein conjugate as described herein and a pharmaceutically acceptable carrier. Related compositions, which are also within the scope of the present invention, include precursors to these pharmaceutical compositions, such as concentrated stock solutions, lyophilized preparations, compositions preserved in frozen form (*e.g.,* with cryoprotective agents), or other compositions that require some manipulation (*e.g.,* dilution, resuspension, purification, and/or sterilization) prior to administration to a patient. These compositions and the pharmaceutically acceptable carriers they may include are further described below. The compositions may be formulated in various ways, including for oral or parenteral (*e.g.,* intravenous or transmucosal) administration.

In a third aspect, the invention features conjugates for use in methods of treating a patient who is suffering from a cancer of the central nervous system or a neurological disorder with CNS involvement (*e.g.,* a disorder associated with inflammation). The methods can include the step of administering to the patient a therapeutically effective amount of a pharmaceutical composition comprising a protein conjugate as described herein. In any of the methods, one can also perform the step of identifying a patient in need of treatment prior to administering the pharmaceutical composition. For example, one can identify a patient who is suffering from a cancer of the CNS by performing one or more diagnostic tests, such as diagnostic imaging. Similarly, one can perform an examination suitable for diagnosing a neurological disorder. Although the invention is not so limited, we clearly contemplate the conjugates for use in the treatment of human patients. Veterinary use *(e.g.,* to treat non-human mammals, such as domesticated pets) is certainly possible, although the cost is likely to remain prohibitively high at least in the near future. While we tend to use the term "patient," we may also refer without meaningful difference to an "individual" or a "subject," whether in the context of human or veterinary use. We use the term "disorder" broadly to refer to any clinically undesirable condition affecting a patient. For example, although some conditions are specifically referred to as diseases (*e.g.,* Parkinson's disease and Alzheimer's disease), these conditions are clinically undesirable and are "disorders" as that term is used herein. Thus, a neurological disorder with CNS involvement can be any such condition typically referred to as a disorder, disease, condition, syndrome, or the like. A "therapeutically effective amount" of a pharmaceutical composition is an amount sufficient to bring about a positive clinical outcome, whether or not that outcome is an eradication of the disorder. Thus, "treating" a patient is accomplished by improving a sign or symptom of the disorder in question or inhibiting or slowing the progression of the disorder to a more severe state. The amount of the protein conjugate administered may be an "equivalent dosage," in which case the amount of the antibody moiety that is administered within the protein conjugate is the same or about the same *(e.g.,* ± 10-20%) as the amount of the antibody moiety that would have been administered to effectively treat a tissue that is not protected by the BBB. Where the disorder is a cancer affecting the CNS, the cancer may be one that arises within the CNS or one that has metastasized to the CNS.

In a fourth aspect, the present invention features methods of making the protein conjugates described herein. The methods are carried out by conjugating an antibody moiety to a polypeptide via a linker. In one embodiment, the method conjugates an antibody moiety to a polypeptide comprising the amino acid sequence Thr₁-Phe₂-Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:117), or a biologically active analog or fragment thereof. In other embodiments, any of the other polypeptides described herein can be incorporated. The methods can include the steps of: (a) providing a linker-bound antibody moiety; (b) providing the polypeptide in modified form; and (c) exposing the linker-bound antibody moiety to the polypeptide in modified form under conditions that produce a protein conjugate comprising the antibody moiety, the linker, and the polypeptide. The polypeptide is in a modified form when it includes a chemical entity reactive with the linker (*e.g.,* a primary amine (*e.g.,* N₃)). The linker-bound antibody moiety can be formed by performing the steps: (a) providing the antibody moiety; and (b) exposing the antibody moiety to a linker under conditions that produce a linker-bound antibody moiety. The modified polypeptide can be formed by performing the steps: (a) providing the polypeptide; and (b) exposing the polypeptide to a chemical entity reactive with the linker under conditions that produce a modified polypeptide.

In another conjugation scheme, the linker is first attached to the polypeptide, and that modified intermediate (a linker-bound polypeptide) is then conjugated to the antibody moiety. In instances where conjugation is mediated by an azide-alkyne cycloaddition reaction, the alkyne within a given linker reacts with an azide on the conjugation partner. For example, where the polypeptide bound to a linker including an alkyne, the antibody moiety can be modified to include an azide (the linker-bound polypeptide and the azide-bearing antibody being conjugation partners). The alkyl and azide groups react to produce a stable triazole. For example, a polypeptide modified by binding to an alkyl-containing dibenzocyclooctyl (DBCO linker) can react with an azide-bearing antibody moiety to produce an stable triazole, which may also be referred to as the Cu(I)-free or strain-promoted click reaction. *See* Baskin et. al., Proc. Natl. Acad. Sci. USA 104(43):16793-16797, 2007; Ning et al. Anweg. Chem. Int. Ed., 47:2253-5; and Jewett et al., J. Am. Chem. Soc., 132(11):3688-3690, 2010.

Should one of ordinary skill in the art desire additional information about conjugation methods and mechanics, one could consult Jewett and Bertozzi ("Cu-free click cycloaddition reactions in chemical biology," Chemical Society Reviews 39:1272-1279, 2010), WO 2012/134925, and U.S. Application Publication Nos. 2005-0222427 and 2009-004753.

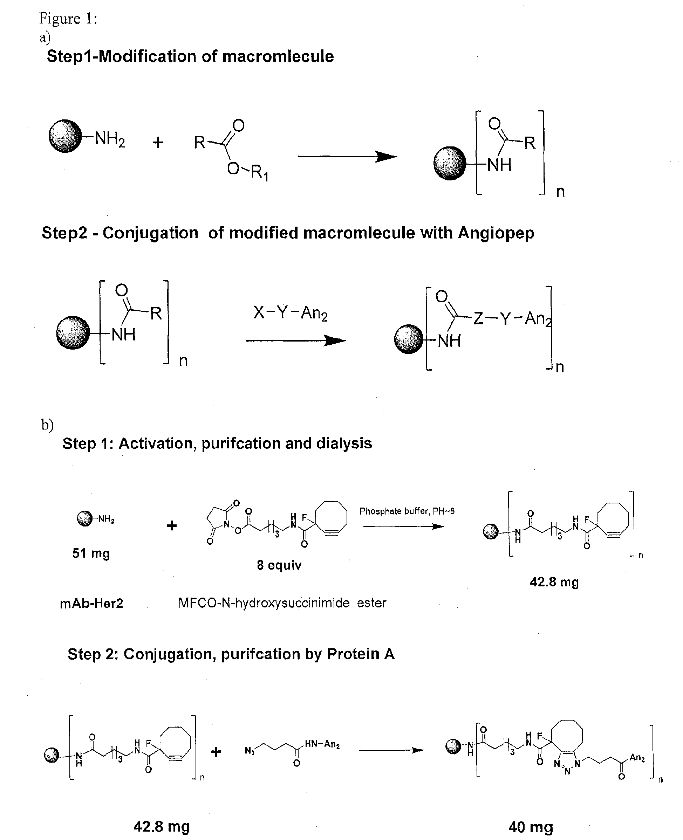

In the methods that involve a linker, the linker can include a cyclic octyne or strained cyclooctyne. The chemical entity on the polypeptide that reacts with the linker can include an azide group. To effect conjugation, the azide group on the modified polypeptide reacts with the octyne on the linker as shown in Figure 1. In Figure 1, the exemplified linker is MFCO-N-hydroxysuccinimide ester. More generally, the linker employed can be a monofluoro cyclooctyne (MFCO), bicyclo[6.1.0]nonyne (BCN), or dibenzocyclooctyne (DBCO). While the conditions for making a protein conjugate are described more fully below, we note here that the linker-bound antibody moiety can be exposed to a modified polypeptide in a light-restricted environment for about 8-24 hours at room temperature. Conjugation can be monitored by monitoring consumption of the modified polypeptide by, for example, liquid chromatography-mass spectrometry (LC-MS). Following conjugation, the protein conjugates can be purified by, for example, column chromatography. For example, one can pass the conjugate over a Protein A column and elute it with a citric acid buffer of approximately pH 3.0.

The linker-bound antibody moieties and the modified polypeptides, which may also be linker-bound, described herein are also compositions of the invention and may be included in the kits of the invention. Thus, in a fifth aspect, the invention features kits. In one embodiment, the kit includes a pharmaceutical composition or a precursor thereto that is packaged together with instructions for use and, optionally, any device useful in manipulating the compositions in preparation for administration and/or in administering the compositions. For example, the kits of the invention can include one or more of: diluents, gloves, vials or other containers, pipettes, needles, syringes, tubing, stands, spatulas, sterile cloths or drapes, positive and/or negative controls, and the like. In another embodiment, the kits of the invention include compositions and reagents useful in making a protein conjugate. For example, the kits can include one or more of: an antibody moiety, a linker, a linker-bound antibody moiety, a polypeptide, a chemical entity reactive with the linker, and/or a modified polypeptide. For example, in one embodiment, a kit can include an antibody moiety, a linker, a chemical entity reactive with the linker, and a polypeptide. In other embodiment, the kit can include a linker-bound antibody moiety and a modified polypeptide. As with the kits useful in administering the compositions, kits useful in making the compositions can include any one or more of: diluents, gloves, vials or other containers, pipettes, needles, syringes, tubing, stands, spatulas, sterile cloths or drapes, positive and/or negative controls, and the like. Any reagents useful in binding a linker to an antibody moiety, modifying a polypeptide, conjugating a linker-bound antibody moiety to a modified polypeptide, or purifying and testing a protein conjugate can also be included. As noted, the linker-bound antibody moieties and modified polypeptides are featured compositions of the invention.

Also disclosed herein are methods of imaging a biological tissue, such as a tissue within the central nervous system. The methods are carried out by providing a conjugate as described herein that has been engineered to include a detectable imaging agent. This agent can vary and can be selected from any of the currently available labels and tags used in imaging. For example, the imaging agent can be, or can include, a fluorescent dye or radioisotope. The imaging agent is incorporated into the present conjugates (*e.g.,* by conventional chemical reactions), which are then administered to a subject. For example, a conjugate comprising a detectable label or tag can be administered intravenously, and the subject can then be examined with a device configured to detect the particular imaging agent that has been incorporated into the conjugate.

In addition to BBB permeability, the protein conjugates of the invention may have one or more of the following advantages: little or no precipitation at dosing concentrations; freeze-thaw stability; and solubility (producing few if any aggregates in solution). The antibody moiety can also retain high affinity for its target (relative to the affinity in an unconjugated form). For example, the antibody moiety can have an affinity for its target that is within about 3-fold of the affinity of the moiety when unconjugated. In studies with the protein conjugate H43, we observed an affinity of 2.2 nM where the affinity of the unconjugated antibody moiety was 1.3 nM. Other features and advantages of the present compositions and methods are illustrated in the description below, the drawings, and the claims.

### BRIEF DESCRIPTION OF THE DRAWINGS

Figures 1a and 1b are schematics illustrating methods of attaching an antibody moiety to a polypeptide using a copper-free "click chemistry" technique. Panel a) depicts a general reaction scheme and panel b) depicts a more specific conjugation process.
Figure 2 is a Table listing polypeptide sequences described and referenced herein.

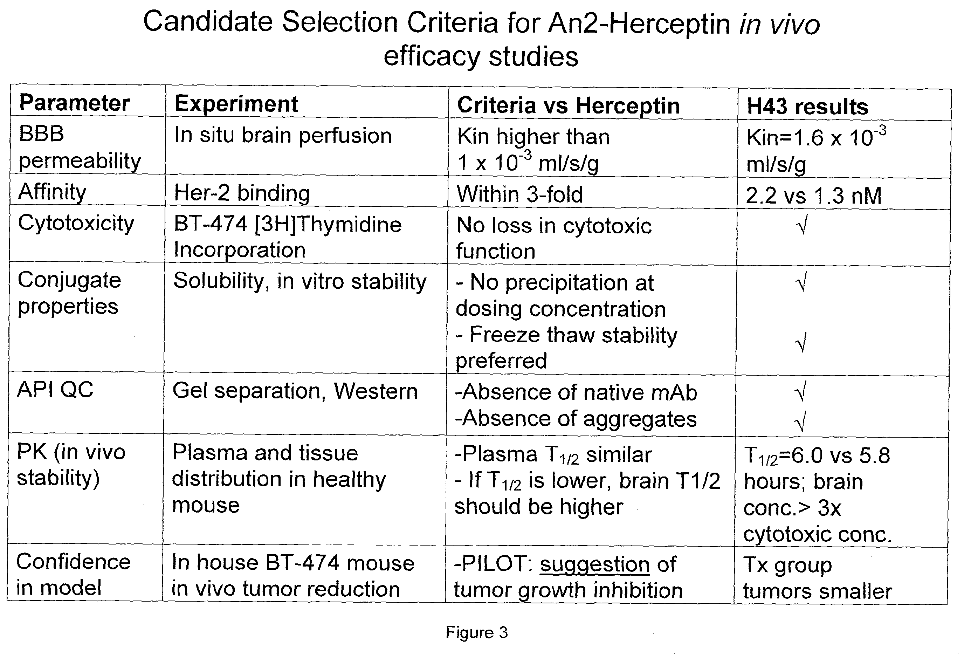

Figure 3 is a chart summarizing certain parameters that may be examined and characterized with respect to the protein conjugates of the invention (*e.g.,* BBB permeability and binding affinity), experiments that can be performed to test these parameters, and the results obtained with an exemplified protein conjugate (H43, comprising an angiopep-2 polypeptide and trastuzumab).
Figure 4 is a bar graph showing the results of a perfusion study with the protein conjugates designated H1, H2, and H3. Vd (ml/100 g/2 min) is shown for each conjugate and for trastuzumab (anti-HER2) in the brain, capillaries, and parenchyma.
Figure 5 is a pair of line graphs showing the results of binding studies with the protein conjugates designated H40 and H41 (left-hand graph) and H42, H43, and H44 (right-hand graph) relative to unconjugated trastuzumab (αHer-2).
Figure 6 is a bar graph showing the results of a perfusion study with the protein conjugates designated H40, H41, H42, H43, and H44. Vd (ml/100 g/2 min) is shown for each conjugate and for trastuzumab (anti-HER2) in the brain, capillaries, and parenchyma.
Figure 7 is a bar graph showing the amount of trastuzumab and a protein conjugate of the invention including trastuzumab and An2 (brain to blood ratio (%)) in the brain tissue of mice at various times after intravenous injection.
Figure 8 is a bar graph showing the results of an *in vivo* study in which mice were injected with BT-474 tumor cells and then treated with a vehicle/control solution, with trastuzumab, or with a protein conjugate of the invention (H43).
Figure 9 is a series of bar graphs showing the results of an *in vivo* study of tissue distribution. The distribution of an unconjugated anti-HER2 antibody and of the conjugated construct H43 is expressed as % [Brain]/[Plasma] (left-hand graph) and as % injected dose/g of tissue (center graph). Plasma levels are shown in the right-hand graph. In each graph, the data obtained with the unconjugated anti-HER2 antibody is represented by the darker, left-most bar of each pair, and the data obtained with H43 is represented by the lighter, right-most bars.
Figure 10 is an alignment of a human aprotinin and AngioPep-2.
The Figure discloses SEQ ID NOS 128 and 129, respectively, in order of appearance.

Figure 11 is a series of four Tables including exemplary linkers for use in the present conjugates and inclusion in the kits of the invention. The first Table lists amine-to-amine linkers, including NHS esters, imidoesters, and others; the second Table lists sulfhydryl-to-sulfhydryl linkers, such as the maleimides and pyridyldithiols; the third Table lists amine-to-sulfhydryl linkers, which includes NHS ester/maliemide compounds. The linkers falling within the scope of the invention are defined in the claims. Examples of these compounds are provided in the Table below.

### DETAILED DESCRIPTION OF THE INVENTION

The majority of the drugs that are potentially useful in treating the CNS do not cross the BBB, and it is surprising that so little effort has been focused on this longstanding problem. It is estimated that more than 99% of CNS drug development worldwide is devoted to drug discovery *per se,* with less than 1% of the effort directed to improving delivery. There are no reliably effective treatments for many serious, costly, and debilitating CNS disorders, including Parkinson's disease, Alzheimer's disease, and brain cancers. Thus, there is a substantial and unmet need for not only discovering effective agents, but also for successfully delivering those agents to the brain and spinal cord.

Therapeutic antibodies are among the most promising new treatments for many types of disorders, including disorders affecting the CNS. Due to their size, therapeutic antibodies are also among the most difficult agents to deliver to the CNS. In the context of the present invention, an antibody moiety, when conjugated to a polypeptide as described herein *(e.g.,* an aprotinin-derived polypeptide), crosses the blood-brain barrier to a greater extent than that same antibody moiety would have crossed the blood-brain barrier alone *(i.e.,* when unconjugated). Differences in transport can also be observed in *ex vivo* models of the BBB. Thus, the polypeptides in the present conjugates are useful in transporting the antibody moiety across the blood-brain barrier of an individual, and the resulting, improved access to the CNS allows the antibody moiety to be used in the treatment and diagnosis of cancers and neurological disorders in ways not previously possible.

The present invention is based, in part, on the inventors work with physiologic-based strategies for drug delivery in which a therapeutic agent (in the present invention, an antibody moiety) is conjugated in a particular manner to a polypeptide (*e.g.,* an aprotinin-derived polypeptide) that serves as a vector or receptor-targeted delivery vehicle with respect to the BBB.

Transporters and receptors present at the BBB are important in maintaining the integrity of the BBB and homeostasis. One family of receptors, the low-density lipoprotein receptor-related proteins (LRPs), which include LRP1 and LRP2 (also known as megalin), mediate the transport of ligands across endothelial cells of the BBB (Shibata *et al.,* 2000; Ito *et al.,* 2006; Bell *et al.,* 2007). More specifically, LRP2 acts as a multi-ligand binding receptor at the plasma membrane of epithelial cells and mediates endocytosis of ligands leading to transcytosis. For example, both LRP1 and LRP2 mediate the endocytosis of secreted-amyloid precursor protein (APP) (Kounnas *et al.,* 1995; Cam and Bu, Mol. Neurodegen. 1:8, 2006). Interestingly, APP lacks the Kunitz protease inhibitor (KPI) domain and is a poor substrate/ligand for an LRP. This suggests that this domain is important for the recognition, internalization, and clearance of APP by LRP (Kounnas *et al.,* 1995).

Aprotinin is a protease inhibitor of the Kunitz-type *(i.e.,* it contains a KPI domain), and it is a ligand for LRP1 and LRP2. It is a biologically active, monomeric polypeptide derived from bovine lung tissue, and it is used to reduce bleeding during complex surgeries. Aprotinin consists of 58 amino acid residues, with ten positively-charged lysine and arginine residues and only four negatively-charged aspartate and glutamate residues. Aprotinin is stable due to the inclusion of three disulfide bonds linking six cysteine residues within the protein. It also contains a twisted β-hairpin and a C-terminal α-helix. *In vitro* studies have demonstrated that aprotinin crosses a cell layer mimicking the mammalian BBB and thus acts as a vector for transporting agents across the BBB of an individual in need.

Accordingly, polypeptides useful in the present conjugates can have one or more of the characteristics of an aprotinin polypeptide or another KPI domain polypeptide. That is, a polypeptide incorporated in the present conjugates can include or consist of about 58 amino acid residues (*e.g.,* 56-60 amino acid residues, inclusive) that exhibit a degree of homology or identity to an aprotinin sequence (*e.g.,* 70-100% homology or identity); can include about ten lysine and/or arginine residues; can contain no more than about four negatively-charged residues (*e.g.,* no more than about four aspartate or glutamate residues); can include about three *(e.g.,* 2-4, inclusive) intramolecular (*e.g.,* disulfide) bonds; and/or can include a twisted β-hairpin and/or C-terminal α helix. The inventors have identified a family of polypeptides that differ from, but retain some degree of similarity to, an aprotinin polypeptide. These aprotinin-derived polypeptides are described herein in the context of newly configured protein conjugates, of which they can be a part. Accordingly, the present conjugates can include an aprotinin sequence described herein (*e.g.,* a polypeptide having a sequence consisting of SEQ ID NO:98 or SEQ ID NO:126 (*see also* Siekmann et al., Biol. Chem. 369(3):157-163, 1988). Although the exact molecular mechanism of transcytosis is unclear, and while the invention is not limited to protein conjugates that function by any particular molecular mechanism, the polypeptides described herein, including aprotinin and aprotinin-derived polypeptides, are thought to interact with a receptor in the LRP family.

***Polypeptides:*** Polypeptides useful in the context of the present conjugates include those having a Kunitz domain, which is understood in the art to inhibit protein degrading enzymes. The polypeptides incorporated in the present conjugates may or may not retain that function. As the domains are typically about 50-60 amino acid residues in length and typically have a molecular weight of about 6 kDa, the polypeptide portion of the present conjugates can have these same characteristics. More specifically, the polypeptide can be aprotinin, Alzheimer's amyloid precursor protein (APP), or tissue factor pathway inhibitor (TFPI). As these proteins may be immunogenic, the invention encompasses less immunogenic fragments of these proteins that chaperone a conjugated antibody moiety across the blood-brain barrier. APP or a biologically active fragment thereof can be used in the present conjugates despite the lack of a KPI domain.

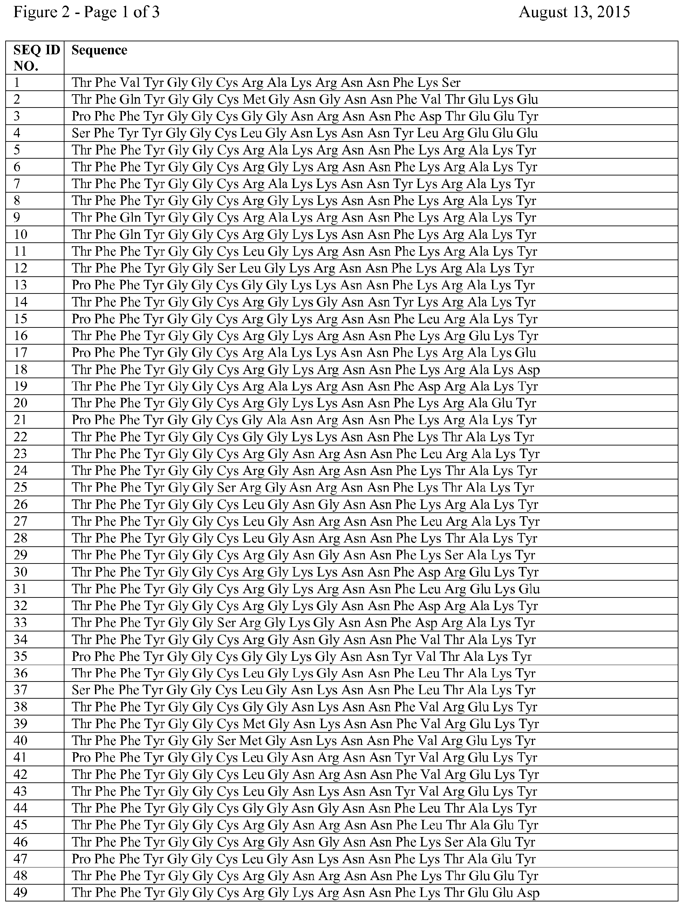

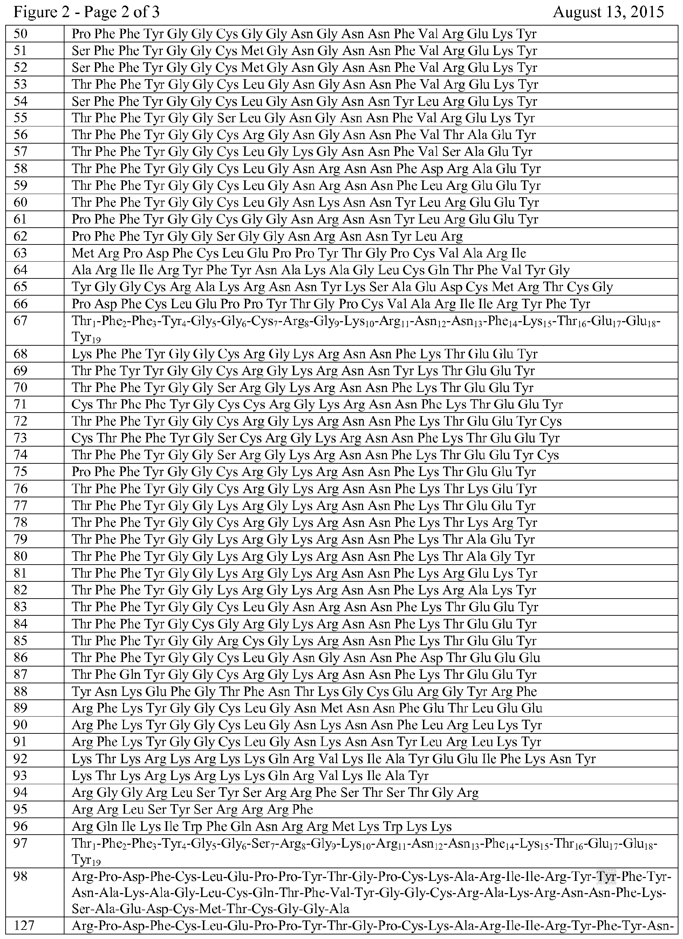

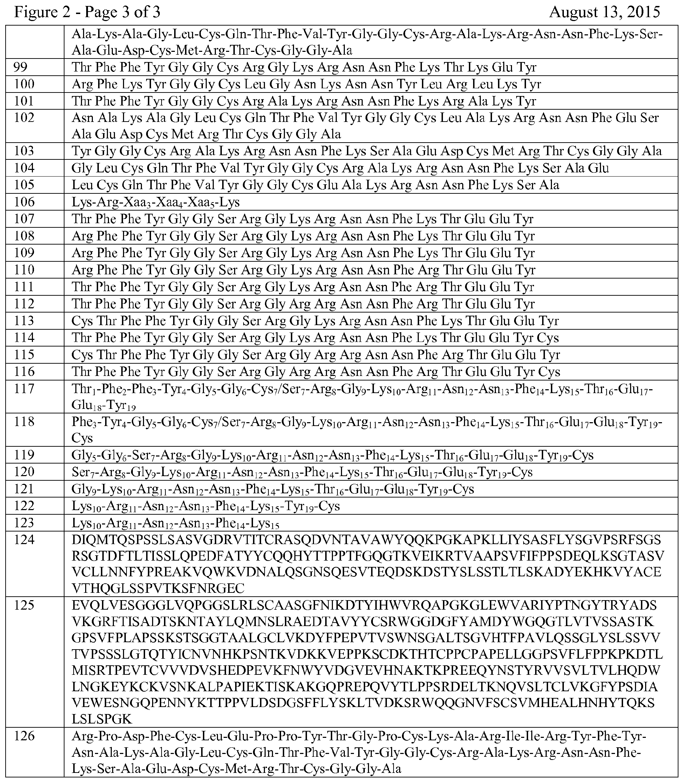

In one embodiment, the polypeptide(s) incorporated in the protein conjugates of the invention are: an aprotinin (*e.g.,* SEQ ID NO:98); a biologically active fragment or analog of aprotinin (this fragment may include one or more of the features of a Kunitz domain, as discussed above). In one embodiment, the polypeptide is represented by SEQ ID NO:117 or is a biologically active fragment or analog of SEQ ID NO: 1 17 *(e.g.,* a fragment or analog shown in the Table of Figure 2).

The amino acid residues within the polypeptides can be of the standard α-amino acid form and can be in the D-form, the L-form, or a mixture of these two enantiomeric forms. As is known in the art, all α-amino acids except glycine can exist in either of two enantiomeric forms, and either or both forms can be incorporated in the present polypeptides as well as in the antibody moieties of the protein conjugates. As is also known in the art, the convention of referring to amino acid residues as having a D- or an L-form is a reference not to the optical activity of the amino acid residue itself, but rather to the optical activity of the isomer of glyceraldehyde from which that amino acid can, in theory, be synthesized (D-glyceraldehyde is dextrorotary and L-glyceraldehyde is levorotatory). Substituting a D-form amino acid residue in the place of an L-form amino acid residue may generate a more stable polypeptide. For example, employing D-lysine in place of L-lysine at position 10 and/or position 15 (or comparable positions in an analog of an aprotinin-derived polypeptide) may increase the stability of an aprotinin-derived polypeptide. Similarly, employing a D-form amino acid at the N-terminal and/or C-terminal of such a polypeptide should increase *in vivo* stability because peptidases cannot utilize a D-amino acid as a substrate (Powell et al., Pharm. Res. 10:1268-1273, 1993). The polypeptides can also be configured as reverse-D polypeptides, which contain D-form amino acid residues arranged in a reverse sequence relative to a polypeptide containing L-amino acids; the C-terminal residue of an L-amino acid polypeptide becomes N-terminal for the D-amino acid polypeptide, and vice versa. Reverse D-polypeptides retain the same tertiary conformation and therefore the same activity as the corresponding L-amino acid polypeptide, but their increased stability can lead to greater therapeutic efficacy (Brady and Dodson, Nature 368:692-693, 1994 and Jameson et al., Nature 368:744-746, 1994). The polypeptides may also be "constrained" by, for example, the addition of cysteine residues that form disulfide bridges (*see* Rizo et al., Ann. Rev. Biochem. 61:387-418, 1992). In any embodiment, the polypeptide can be a cyclic polypeptide (*e.g.,* cyclized by the inclusion of terminal cysteine residues that form disulfide bonds).

With respect to their preparation, polypeptides useful in protein conjugates can be produced by any methods known in the art, including by synthetic methods and recombinant techniques used routinely to produce proteins from nucleic acids. The resulting polypeptides may be stored in an unpurified or in an isolated or substantially purified form until further use. For example, the polypeptides can be stored until used in the methods described below for generating a protein conjugate of the invention. Chemical synthesis can be achieved, for example, by solid phase synthesis, and recombinant techniques can include expression from vector constructs in a biological cell *(e.g.,* a prokaryotic or eukaryotic cell). Codons that encode specific amino acid residues are well known in the art (*see, e.g.,* "Biochemistry," 3rd Ed., 1988, Lubert Stryer, Stanford University, W.H. Freeman and Company, New-York), as are methods for codon optimization. An exemplary nucleotide sequence encoding an aprotinin analogue is illustrated in SEQ ID NO:106 and may be found in GenBank under Accession No. X04666. This sequence encodes an aprotinin analogue having a lysine at position 16 (with reference to the amino acid sequence encoded by SEQ ID NO:106) instead of a valine as found in SEQ ID N0:98. A mutation in the nucleotide sequence of SEQ ID NO:106 may be introduced by methods known in the art to produce the peptide of SEQ ID N0:98 having a valine in position 16.

The polypeptides described herein for inclusion in a protein conjugate may be post-translationally modified, either within a cell in which the polypeptide is expressed or *ex vivo* by chemical modification. For example, a polypeptide as described herein can be pegylated, acetylated, acylated, amidated, oxidized, cyclized, and/or sulfonated.

As described at length, the polypeptides within the conjugates may have a sequence as specifically set out herein *(e.g.,* the sequence represented by SEQ ID NO:67 or 97 or any other aprotinin-derived polypeptide shown in the Table of Figure 2) or they may be biologically active fragments or analogs of any of these reference sequences. A fragment differs from a reference sequence by virtue of having fewer contiguous amino acid residues (one or more amino acids from the N- or C-terminal are deleted) whereas an analog differs from the reference sequence by virtue of including at least one additional amino acid residue or at least one amino acid substitution. The additional amino acid residue(s) can be added to the N-terminal, the C-terminal, to a position between the N- and C-termini of a reference polypeptide (*e.g.,* SEQ ID NO:117), or at any combination of these positions. An analog may be shorter than a reference sequence *(i.e.,* it may include a deletion of one or more amino acid residues), however, we use the term "analog" to mean a variant that differs in some way from the reference sequence other than just a simple deletion of an N- and/or C-terminal amino acid residue or residues. Where the analog includes a substitution of an amino acid residue, the substitution may be considered conservative or non-conservative. Conservative substitutions are those within the following groups: Ser, Thr, and Cys; Leu, Ile, and Val; Glu and Asp; Lys and Arg; Phe, Tyr, and Trp; and Gln, Asn, Glu, Asp, and His. For example, replacing a serine residue with threonine or cysteine would be considered a conservative amino acid substitution; replacing leucine with isoleucine or valine would be considered a conservative amino acid substitution; and so forth. Conservative substitutions may also be defined by the BLAST (Basic Local Alignment Search Tool) algorithm, the BLOSUM substitution matrix (*e.g.,* BLOSUM 62 matrix), or the PAM substitution:p matrix (*e.g.,* the PAM 250 matrix).

Where a reference sequence has 19 amino acid residues, a biologically active fragment thereof may have 6-18 contiguous residues (inclusive). Where the reference sequence has 19 amino acid residues, a biologically active analog thereof may have 1-13 amino acid substitutions; one or more amino acid additions (*e.g.,* the addition of residues at the N- and/or the C-terminal that increase the length of the reference polypeptide to up to about 50 amino acid residues); or a combination of such substitutions and additions. As noted, in the case of an analog (as opposed to a fragment), where there is at least one substitution and/or at least one addition, there may also be at least one deletion.

The degree of similarity between a biologically active fragment or analog of an aprotinin-derived polypeptide and the reference polypeptide may also be expressed as the percentage of the residues that are identical at comparable positions. A biologically active fragment or analog of an aprotinin-derived polypeptide may be at least 35%, 40%, 50%, 60%, 70%, 80%, 90%, 95%, or 98% identical to a reference polypeptide (*e.g.,* an aprotinin-derived polypeptide assigned a sequence identifier herein).

Just as the enantiomeric form of the amino acid residues incorporated can alter the stability of a polypeptide, one can modify the length and content of the polypeptide *(e.g.,* an aprotinin-derived polypeptide) to optimize a characteristic such as charge or polarity, hydrophilicity or hydrophobicity, bioavailability, and conjugation properties. For example, one can promote a positive charge by deleting one or more amino acids (*e.g.,* from 1 to about 3 amino acid residues) that are not basic/positively charged or that are less positively charged *(e.g.,* as determined by pKa). Alternatively, or in addition, positive charge can be promoted by adding one or more amino acids *(e.g.,* from 1 to about 3 amino acid residues) that are basic/positively charged or more positively charged *(e.g.,* as determined by pKa) than the residues they replace. One of ordinary skill in the art would recognize that naturally occurring residues have recognized and shared properties, largely attributed to the properties of their side chains. The following information can be used as desired to modify the properties of the aprotinin-derived polypeptides. To increase hydrophobicity, norleucine, methionine (Met), alanine (Ala), valine (Val), leucine (Leu), isoleucine (Ile), histidine (His), tryptophan (Trp), tyrosine (Tyr), and phenylalanine (Phe) can be incorporated; to increase neutrality or hydrophilicity, cysteine (Cys), serine (Ser), and threonine (Thr) can be incorporated; to increase acidity or negative charge, aspartic acid (Asp) or glutamic acid (Glu) can be incorporated; to promote basicity, asparagine (Asn), glutamine (Gln), histidine (His), lysine (Lys), and arginine (Arg) can be incorporated. Residues that influence chain orientation include glycine (Gly) and proline (Pro). Residues with aromatic side chains include tryptophan (Trp), tyrosine (Tyr), phenylalanine (Phe), and histidine (His).

In the reference sequences provided herein, the amino acid residues are naturally occurring. Biologically active analogs of these sequence can, however, include one or more non-naturally occurring residues. For example, they may include selenocysteine (*e.g.,* seleno-L-cysteine) at any position, including in the place of cysteine at position 7. Many other "unnatural" amino acid substitutes are known in the art and are available from commercial sources such as the Sigma Aldrich chemical company. Examples of non-naturally occurring amino acids include D-amino acids in most instances, amino acid residues having an acetylaminomethyl group attached to a sulfur atom of a cysteine, a pegylated amino acid, and omega amino acids of the formula NH₂(CH₂)ₙCOOH wherein n is 2-6 neutral, nonpolar amino acids, such as sarcosine, t-butyl alanine, t-butyl glycine, N-methyl isoleucine, and norleucine. Phenylglycine may substitute for Trp, Tyr, or Phe; citrulline and methionine sulfoxide are neutral nonpolar, cysteic acid is acidic, and ornithine is basic. Proline may be substituted with hydroxyproline and retain the conformation conferring properties of proline. The protein conjugates of the invention encompasses polypeptides and antibody moieties including such residues. It is to be understood that where we refer to a modification or feature such as the inclusion of various enantiomers, a post-translational modification, or the inclusion of an "unnatural" residue, that modification or feature may be present in the part of the conjugate described herein as the aprotinin-derived polypeptide, in the part of the conjugated described herein as the antibody moiety, or in any other amino acid-containing part of the conjugate (*e.g.,* in a detectable label).

Regardless of precise sequence or characteristics of a biologically active fragment or analog of a referenced polypeptide, that variant may have either a reduced ability or an enhanced ability to transport an antibody moiety across the BBB relative to the ability of a referenced sequence. Of course, a referenced sequence and a biologically active fragment or analog thereof may also have about the same abilities with regard to BBB transport. For example, where the polypeptide is a biologically active variant of a referenced polypeptide *(e.g.,* of SEQ ID NO:67, 97, or 117), the ability of the variant to transport a conjugated antibody moiety may be reduced, relative to the referenced polypeptide, by at least or about 5% *(e.g.,* by at least or about 5%, 10%, 20%, 25%, 35%, 50%, 60%, 70%, 75%, 80%, 90%, or 95%). Where the polypeptide is a biologically active variant of a referenced polypeptide *(e.g.,* of SEQ ID NO:67, 97, or 117), the ability of the variant to transport a conjugated antibody moiety may be enhanced, relative to the referenced polypeptide, by at least or about 5% *(e.g.,* by at least or about 5%, 10%, 25%, 50%, 100%, 200%, 500%, or 1000%). As noted above, while activity may vary, a biologically active fragment or analog of a referenced polypeptide is one that is active enough to achieve a beneficial result *(e.g.,* a clinically beneficial result in a patient or, on average, a group of patients to whom it is administered). The beneficial result may be a beneficial treatment or diagnostic procedure.

In particular embodiments, the polypeptide can have the sequence: Thr₁-Phe₂-Phe₃-Tyr₄-Gly₅-Gly₆-Cys₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:67) or Thr₁-Phe₂-Phe₃-Tyr₄-Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:97). As noted above, the polypeptide can be a fragment or an analog of SEQ ID NO:117 in which at least 13 of the 19 amino acid residues of SEQ ID NO:117 remain invariant. In particular, the analog of SEQ ID NO:117 can include a sequence in which K₁₀ and/or K₁₅ remain invariant. In some embodiments, Asn₁₂ is substituted with another amino acid residue *(e.g.,* Gln); Asn₁₃ is substituted with another amino acid residue *(e.g.,* Gln); and/or Phe₁₄ is substituted with another amino acid residue (*e.g.,* Tyr or Trp). The polypeptide can include the sequence: Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:118); Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:119); Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:120); Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:121); or Lys₁₀-Arg₁₁-Asn₁₂-Asn₁₃-Phe₁₄-Lys₁₅-Tyr₁₉-Cys (SEQ ID NO:122).

Where the polypeptide is a fragment of SEQ ID NO:117, or where the polypeptide is an analog of SEQ ID NO:117 that includes a fragment of SEQ ID NO:117, the fragment may have a length of at least 6 amino acid residues (*e.g.,* 6, 7, 8, 9, 10, 11, 12, 13, 14, 15, 16, 17, or 18 amino acid residues) that are identical to 6-18 contiguous amino acid residues of SEQ ID NO:117. In accordance with the present invention, the fragment can be Lys₁₀-Arg₁₁-Asn₁₂-Asn₁₃-Phe₁₄-Lys₁₅ (SEQ ID NO:123). Biologically active analogs of SEQ ID NO:17 can have (consist of), or can include (comprise) a sequence in which the lysine residues at positions 10 and 15 are invariant, or a sequence in which the lysine residues at positions 10 and 15 as well as the arginine residue at position 16 is invariant. In the latter case, the biologically active analog would have, or would include, a sequence conforming to the formula Lys-Arg-Xaa₃-Xaa₄-Xaa₅-Lys (Formula I; SEQ ID NO:106). Xaa₃ can be Asn or Gln; Xaa₄ can be Asn or Gln; and Xaa₅ can be Phe, Tyr, or Trp. The sequence noted above, Lys₁₀-Arg₁₁-Asn₁₂-Asn₁₃-Phe₁₄-Lys₁₅ (SEQ ID NO:123) conforms to Formula I. Where Xaa₃, Xaa₄, and Xaa₅ are selected, they can be conservative substitutions *(i.e.,* the contiguous Asn residues can be replaced, independently with Gln, Glu, Asp, or His, and Phe can be replaced with Tyr or Trp). Xaa₃, Xaa₄, and Xaa₅ can also vary in enantiomeric form, can be non-naturally occurring amino acid residues, or can be selected on the basis of another feature or characteristic as described herein.

In particular embodiments, the aprotinin-derived polypeptide has (consists of) or includes (comprises) a sequence shown in the Table of Figure 2, designated as SEQ ID NO:1-SEQ ID NO:123.

***Modified polypeptides:*** Any of the polypeptides incorporated in the protein conjugates of the invention can be modified to chemically interact with a linker *(e.g.,* a linker bound to an antibody moiety) as described further below. These modified polypeptides are within the scope of the present invention and may be packaged as a component of a kit with instructions for completing the process of conjugation to a linker-bound antibody moiety. For example, a polypeptide can be modified to include an N₃ azide group, which will react with an alkyne in a linker bound to an antibody moiety (and *vice versa;* the polypeptide can be bound to linker including an alkyne, which will react with an azide group extending from an antibody moiety).

***Antibody Moieties:*** In addition to a polypeptide *(e.g.,* an aprotinin-derived polypeptide), the protein conjugates of the present invention include a single antibody moiety or a biologically active variant thereof. As noted above, the antibody moiety can be a naturally expressed antibody (*e.g.,* a tetrameric antibody) or a biologically active variant thereof. The antibody moiety can also be a non-naturally occurring antibody (*e.g.,* a single chain antibody) or a biologically active variant thereof. The variants include, without limitation, a fragment of a naturally occurring antibody (*e.g.,* an Fab fragment or an F(ab')2 fragment of, *e.g.,* a tetrameric antibody), a fragment of an scFv, or a variant of a tetrameric antibody, an scFv, or fragments thereof that differ by virtue of the addition and/or substitution of one or more amino acid residues.

As is well known in the art, certain types of antibody fragments can be generated by enzymatic treatment of a "full-length" antibody. Digestion with the enzyme papain produces two identical Fab fragments, each with a single antigen-binding site, and a residual Fc fragment. The Fab fragment also contains the constant domain of the light chain and the C_{H1} domain of the heavy chain. In contrast, digestion with the enzyme pepsin yields the F(ab')₂ fragment that has two antigen-binding sites and is still capable of cross-linking antigen. Antibody moieties incorporated in the present conjugates can be generated by digestion with these enzymes or produced by other methods.

Fab' fragments, which can also be incorporated, differ from Fab fragments in that they include additional residues at the C-terminus of the C_{H1} domain, including one or more cysteine residues from the antibody hinge region. The cysteine residues of the constant domains bear a free thiol group, which can participate in the conjugation reactions described herein..F(ab')₂ antibody fragments are pairs of Fab' fragments linked by cysteine residues in the hinge region. Other chemical couplings of antibody fragments are also known in the art.

The Fv region is a minimal fragment that contains a complete antigen-recognition and binding site consisting of one heavy chain and one light chain variable domain. The three CDRs of each variable domain interact to define an antigen-biding site on the surface of the V_{H}-V_{L} dimer. Collectively, the six CDRs confer antigen-binding specificity to the antibody. As would be known in the art, a "single-chain" antibody or "scFv" fragment is a single chain Fv variant formed when the V_{H} and V_{L} domains of an antibody are included in a single polypeptide chain that recognizes and binds an antigen. Typically, single-chain antibodies include a polypeptide linker between the V_{H} and V_{L} domains that enables the scFv to form a desired three-dimensional structure for antigen binding (*see, e.g.,* Pluckthun, In The Pharmacology of Monoclonal Antibodies, Rosenburg and Moore eds., Springer-Verlag, New York, 113:269-315, 1994).

In other embodiments, the antibody moiety can be a diabody. Diabodies are small antibody fragments that have two antigen-binding sites. Each fragment contains a V_{H} domain concatenated to a V_{L} domain. However, since the linker between the domains is too short to allow pairing between them on the same chain, the linked V_{H}-V_{L} domains are forced to pair with complementary domains of another chain, creating two antigen-binding sites. Diabodies are described more fully, for example, in EP 404,097; WO 93/11161; and Hollinger et al., Proc. Natl. Acad. Sci. USA 90:6444-6448, 1993.

In other embodiment, the antibody moiety can be a linear antibody. In this case, the antibody moiety is formed from a pair of tandem Fd segments (VH-CH1-VH-CH1) that form a pair of antigen binding regions. Linear antibodies can be bispecific or monospecific as described in, for example, Zapata et al. 1995, Protein Eng. 8(10):1057-1062, 1995.

With respect to targets, the antibody moiety *(e.g.,* a tetrameric antibody, a biologically active variant thereof, an scFv, Fab fragment, Fab' fragment, or F(ab')2 fragment, or biologically active variants thereof, regardless of class *(i.e.,* whether of the IgG class or another class) and whether human, humanized, chimeric, polyclonal, monoclonal, or having any other attribute or characteristic described herein) can specifically bind a growth factor receptor or a cytokine receptor *(e.g.,* an interleukin receptor). The growth factor receptor can be a receptor bound by a member of the epidermal growth factor (EGF) family. Examples of receptors for proteins in the EGF family include an EGF receptor (EGFR), a heparin-binding EGF-like growth factor receptor (HB-EGFR), an amphiregulin receptor (AR), an epiregulin receptor (EPR), an epigen receptor, a betacellulin receptor, and a receptor for a neuregulin (*e.g.,* a receptor for neuregulin-1, neuregulin-2, neuregulin-3, or neuregulin-4). In the present invention, the antibody moiety can be trastuzumab, cetuximab, or panitumumab, an scFv comprising the variable regions of the heavy and light chains of trastuzumab, cetuximab, or panitumumab, or a biologically active variant of these tetrameric or single chain antibodies. For example, an Fab or F(ab')2 fragment of trastuzumab, cetuximab, or panitumumab. With respect to function, the antibody moiety can be a chemotherapeutic agent or an anti-inflammatory agent.

Several of our studies to date, including some of those described in the Examples below, have been conducted with trastuzumab (HERCEPTIN®), which is a monoclonal antibody humanized from the mouse that binds with high affinity to the human epidermal growth factor 2 (HER2)/neu receptor. Trastuzumab is FDA approved for the treatment of breast cancer. The HER2/neu receptor is thought to be an orphan receptor with no known ligand. However, it is expressed on the surface of tumor cells and regulates critical cellular processes such as cell cycle progression, cell survival, cell proliferation, and cell motility. Trastuzumab or any antibody moiety that selectively binds HER2 may be a clinically relevant biomarker for certain tumor types. Therapeutically, when trastuzumab binds to the HER receptor, it not only inhibits the cell's ability to grow and divide, but also marks the cell for destruction by the host's immune system.

***Linkers:*** A variety of linkers can be used to generate the present conjugates. For example, an antibody moiety or a biologically active variant thereof can be bound to a linker that reacts with modified free amines, which are present at lysine residues within the polypeptide and at the amino-terminus of the polypeptide. Thus, linkers useful in the present conjugates can comprise a group that is reactive with a primary amine on the polypeptide or modified polypeptide to which the antibody moiety is conjugated. More specifically, the linker is selected from the group consisting of monofluoro cyclooctyne (MFCO), bicyclo[6.1.0]nonyne (BCN), and dibenzocyclooctyne ester (a DBCO ester).

Homobifunctional and heterobifunctional cross-linkers (conjugation agents) are available from many commercial sources. Although known in the art, we note briefly that the reactive groups in homobifunctional crosslinkers are identical and positioned at opposite ends of the crosslinker's spacer arm. They are convenient to work with, as the reaction can be completed with a one-step chemical crosslink, and they can be assembled where desired to form dimers and polymers. Among the commercially available homobifunctional cross-linkers are: BSOCOES (Bis(2-[Succinimidooxycarbonyloxy]ethyl) sulfone; DPDPB (1,4-Di-(3'-[2pyridyldithio]-propionamido) butane; DSS (disuccinimidyl suberate); DST (disuccinimidyl tartrate); Sulfo DST (sulfodisuccinimidyl tartrate); DSP (dithiobis(succinimidyl propionate); DTSSP (3,3'-Dithiobis(sulfosuccinimidyl propionate); EGS (ethylene glycol bis(succinimidyl succinate)); and BASED (Bis(β-[4-azidosalicylamido]-ethyl)disulfide iodinatable).

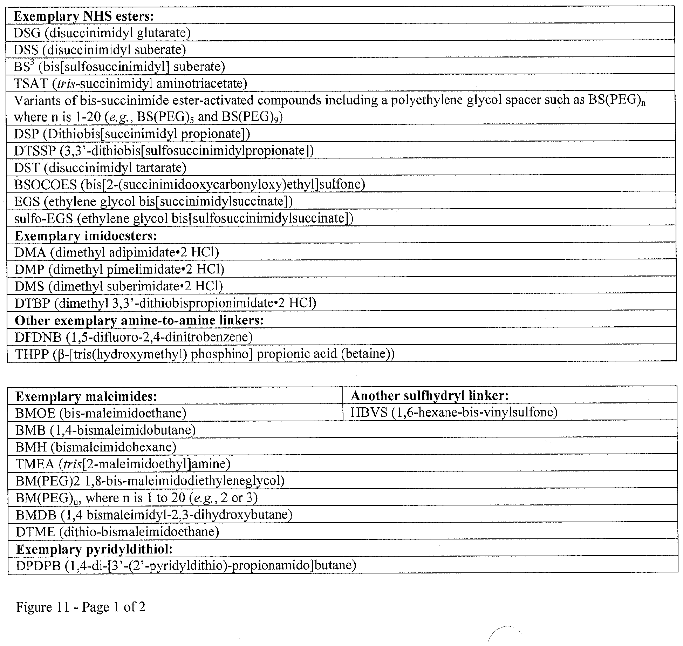

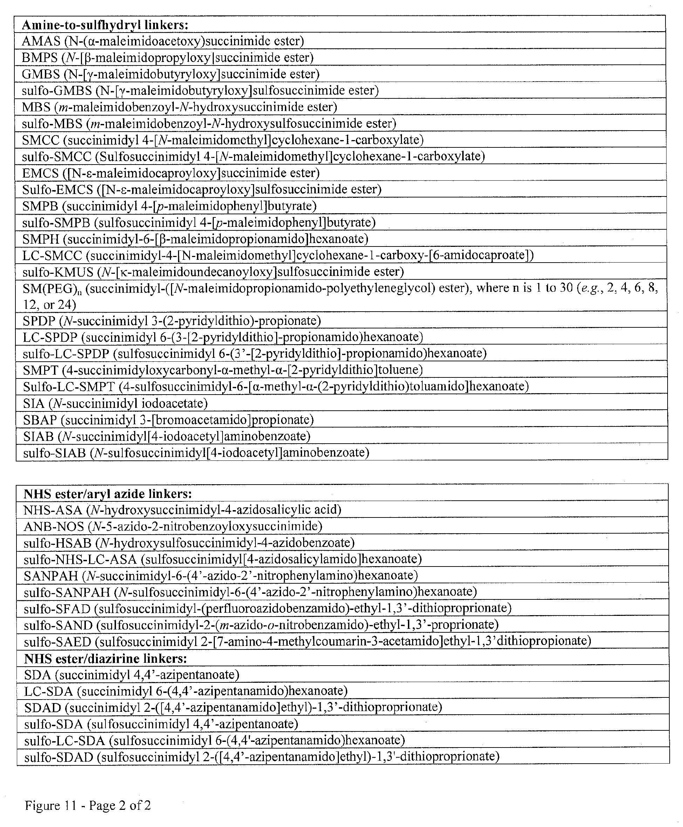

Regions available for cross-linking with a homo- or heterobifunctional cross linker may be found on the polypeptides of the present invention. The cross-linker (or linker, as that term is more commonly used herein) may comprise a flexible arm, such as for example, a short arm (<2 carbon chain), a medium-size arm (from 2-5 carbon chain), or a long arm (3-6 carbon chain). Useful cross-linkers include BS3 ([Bis(sulfosuccinimidyl)suberate], which is a homobifunctional N-hydroxysuccinimide ester that targets accessible primary amines. Another useful linker is NHS/EDC (N-hydroxy-succinimide and N-ethyl-'(dimethylaminopropyl)carbodimide, which allows for the conjugation of primary amine groups with carboxyl groups). Another useful linker is sulfoEMCS ([N-e-Maleimido-caproic acid]hydrazide, which includes heterobifunctional reactive groups (a maleimide and an NHS-ester) that are reactive toward sulfhydryl and amino groups. Another useful linker is hydrazide. Most proteins contain exposed carbohydrates and hydrazide is a useful reagent for linking carboxyl groups to primary amines. Another useful linker is SATA (N-succinimidyl-S-acetylthioacetate). SATA is reactive towards amines and adds protected sulfhydryl groups. With respect to the method used, the process of conjugating an antibody moiety to the polypeptide preferably does not alter or change the key characteristics of the antibody moiety, such as its immunospecificity or immunoreactivity. Homobifunctional amine-specific cross linkers can rely on NHS-ester and imidoester reactive groups for selective conjugation of primary amines and may be cleavable. Heterobifunctional crosslinkers have two distinct groups, which allows the conjugation to progress as a two-step reaction. These linkers are also commercially available in different lengths with different types of spacer arms. Conjugation can proceed between primary amine groups *(e.g.,* on a lysine residue) and sulfhydryl groups *(e.g.,* on a cysteine residue). Useful linkers are listed in the Tables of Figure 11.

***Protein Conjugates and Methods of Making the Same:*** The antibody moiety, linker, and polypeptide within a given conjugate can be selected independently. That is, any of the linkers described herein can be used to conjugate any of the polypeptides described herein to any of the antibody moieties described herein. The antibodies, polypeptides and linkers of the invention are defined in the claims. The conjugates can then be used to deliver any of the antibody moieties described herein to the CNS for therapeutic reasons. With the inclusion of a detectable marker, the present conjugates can also be used as imaging agents, providing the means to map the distribution of antigens to which the antibody moieties bind within the CNS.

The protein conjugates of the invention may be formed by joining *(i.e.,* conjugating), without limitation, one or more polypeptide moieties to an antibody moiety.

Also disclosed are techniques including the cross linker SATA and application of click chemistry. SATA is a heterobifunctional cross linker that facilitates the formation of a covalent bond to link two molecules *(i.e.,* antibody moiety to the polypeptide moiety). The succinimidyl ester reacts with a primary amine to introduce a thiol group into the molecule followed by the removal of the acetyl group, thereby generating a sulfhydryl. The thiol group provides the target to link the two moieties together via a disulfide bond. One of the advantages of applying the SATA reaction is that the modified molecule can be stored for long periods of time for later conjugation reactions because the sulfhydryl groups can be added in a protected form.

As noted, copper-free click chemistry techniques may be used to produce the conjugates described herein. Click chemistry is generally understood as a modular reaction that is widely applicable, stereospecific, and capable of producing high yields of products under mild conditions (*e.g.,* physiological conditions). Click chemistry has been described as encompassing four classes of chemical transformations. The first are non-aldol type carbonyl chemical reactions, such as those that form ureas, thioureas, oxime ethers, hydrazone, amides, and aromatic heterocycles. The second transformations are nucleophilic substitution reactions in which a ring within a strained heterocyclic electrophile (*e.g.,* epoxides, aziridines and aziridinium ions) is opened. In the third, addition reactions to C-C multiples bonds, such as Michael addition, epoxidation, aziridation, and dihydroxylation occurs, and in the fourth, are cycloaddition reactions, such as 1,3-dipolar cycloaddition and Diels-Alder reactions. 1,3-dipolar cycloaddition (1,3-Huisgen reaction) of an alkyne and an azide to form five membered triazole is a particular example of a click reaction. *See,* Guddehalli Parameswarappy, Sharavathi, "Bifunctional cyclooctynes in copper-free click chemistry for applications in radionuclide chemistry nd 4-Alkylpyridine derivatives in intramolecular dearomatization and heterocycle synethsis", Dissertation, University of Iowa, 2011. http://ir.uiowa.edu/etd/2710. Click chemistry methods may utilize the highly toxic catalyst copper in production methods that produce high yields. Preliminary conjugation reactions showed, however, that the overall incorporation of a polypeptide in a conjugate of the invention was very low (6%). The conjugate was also unstable, and a precipitation of the product was observed. Therefore, the conjugation steps using click chemistry were optimized to eliminate the use of copper. Briefly, step one involves the activating groups for conjugation as well as purification and dialysis of the intermediate. For this step, a first component (*e.g.,* the antibody or antibody fragment) reacts with a linker generating a first component-linker intermediate (*e.g.,* an antibody- or antibody fragment-linker intermediate). Next, the linker having an activated group for conjugation *(i.e.,* alkyne) and a second component (*e.g.,* a polypeptide having an azide) react forming the desired conjugate which can be further purified. This second step is efficient because the reaction between the relevant groups (alkyne and azide moieties) takes place within 24 hours at room temperature and without denaturing the protein. Neither step in this procedure interferes with the biological activity of the molecules generated.

Alternatively, the polypeptide moiety may be attached or conjugated to a carbohydrate moiety of the antibody molecule through an oxidation process. The method of carbohydrate oxidation can be chemical or enzymatic. The carbohydrate moiety can be located on the Fc region of the antibody, Fab, or Fab' fragments. Oxidation of the Fc region of the antibody moiety can be carried out using known methods to produce an aldehyde. Oxidizing agents can be selected from the group consisting of periodic acid, paraperiodic acid, sodium metaperiodate and potassium metaperiodate. This step is followed by a reaction with an amine group selected from the group consisting of ammonia derivatives such as primary amine, secondary amine, hydroxylamine, hydrazine, hydrazide, phenylhydrazine, semicarbazide or thiosemicarbazide). The enzymatic method involves reacting the carbohydrate moiety of the antibody molecule with an enzyme such as galactose oxidate in the presence of oxygen to form an aldehyde.

The protein conjugates of the present invention can be assessed in any number of ways. For example, a protein conjugate can be assessed for BBB permeability (by *in situ* brain perfusion or testing in an *ex vivo* model of the BBB such as the model described in U.S. Patent No. 7,557,182); for the affinity of the antibody moiety for its target; for cytotoxicity (*e.g.,* by BT-474 [³H]-thymidine incorporation); for solubility and/or stability *in vitro;* for purity *(e.g.,* low levels of unconjugated antibody moieties and low levels of protein aggregates can be confirmed by gel separation and Western blotting); and for stability and tissue distribution *in vivo* (*e.g.,* by measuring plasma levels over time and tissue distribution by imaging assays).

*Pharmaceutical compositions:* The present invention also features pharmaceutical compositions that contain a therapeutically effective amount of a protein conjugate of the invention. The compositions can be formulated for administration by any of a variety of routes of administration, and can include one or more physiologically acceptable excipients, which may vary depending on the route of administration. We use the term "excipient" broadly to mean any compound or substance, including those that may also be referred to as "carriers" or "diluents." Preparing pharmaceutical and physiologically acceptable compositions is generally considered to be routine in the art, and one of ordinary skill in the art can consult numerous authorities for guidance. For example, one can consult Remington's Pharmaceutical Sciences, Mack Publishing Company, Philadelphia, PA, 17th ed., 1985. For a brief review of methods for drug delivery, see, *e.g*., Langer (Science 249:1527-1533, 1990).

The pharmaceutical compositions of the present invention can be prepared for oral or parenteral administration, although we expect parenteral administration to be favored (not for convenience but to optimize the delivery of the active diagnostic or pharmaceutical agent (here, the antibody moiety)). Pharmaceutical compositions prepared for parenteral administration include those prepared for intravenous (or intra-arterial), intramuscular, subcutaneous, intraperitoneal, transmucosal *(e.g.,* intranasal, intravaginal, or rectal), or transdermal *(e.g.,* topical) administration. These routes can be used for either diagnostic or therapeutic treatment. Aerosol inhalation is also contemplated. Thus, the invention provides compositions for parenteral administration that include protein conjugates dissolved or suspended in an acceptable carrier, preferably an aqueous carrier, such as water, buffered water, saline, buffered saline *(e.g.,* PBS), and the like. One or more of the excipients included may help approximate physiological conditions, such as pH adjusting and buffering agents, tonicity adjusting agents, wetting agents, detergents, and the like. Where the compositions include a solid component (as they may for oral administration), one or more of the excipients can act as a binder or filler *(e.g.,* for the formulation of a tablet, a capsule, and the like). Where the compositions are formulated for application to the skin or to a mucosal surface, one or more of the excipients can be a solvent or emulsifier for the formulation of a cream, an ointment, and the like.

The pharmaceutical compositions may be sterile; they may be sterilized by conventional sterilization techniques or may be sterile filtered. Aqueous solutions may be packaged for use as is, or lyophilized, the lyophilized preparation, which is encompassed by the invention, being combined with a sterile aqueous carrier prior to administration. The pH of the pharmaceutical compositions typically will be between 3 and 11 (*e.g.,* between about 5 and 9) or between 6 and 8 (*e.g.,* between about 7 and 8). In some embodiments, the pH of the pharmaceutical compositions is between about 7.0 and 7.5. The resulting compositions in solid form may be packaged in multiple single dose units, each containing a fixed amount of the above-mentioned agent or agents, such as in a sealed package of tablets or capsules. The composition in solid form can also be packaged in a container for a flexible quantity, such as in a squeezable tube designed for a topically applicable cream or ointment.

*Methods of treatment and Use:* The pharmaceutical compositions described above can be formulated to include a diagnostically useful or therapeutically effective amount of a protein conjugate. Therapeutic administration encompasses prophylactic applications, and any of the therapeutic methods described herein can be expressed in terms of the "use" of the protein conjugate *(e.g.,* in the treatment of cancer or in the preparation of a medicament useful in treating cancer). Based on genetic testing and other prognostic methods, a physician in consultation with their patient may choose a prophylactic administration where the patient has a clinically determined predisposition or increased susceptibility (in some cases, a greatly increased susceptibility) to a CNS cancer or neurological disorder with CNS involvement. The pharmaceutical compositions of the invention can be administered to the subject (*e.g.,* a human patient) in an amount sufficient to delay, reduce, or preferably prevent the onset of clinical disease. In therapeutic applications, compositions are administered to a subject (*e.g.,* a human patient) already suffering from a CNS cancer or a neurological disorder with CNS involvement in an amount sufficient to at least partially improve a sign or symptom or to inhibit the progression of (and preferably arrest) the symptoms of the condition, its complications, and consequences. An amount adequate to accomplish this purpose is defined as a "therapeutically effective amount." For example, in the treatment of a neurological disorder (*e.g.,* Parkinson's Disease, Alzheimer's Disease, or Huntington's Disease), an agent or compound that decreases, prevents, delays, suppresses, or arrests any symptom of the disorder would be therapeutically effective. A therapeutically effective amount of a pharmaceutical composition may be an amount that achieves a cure, but that outcome is only one among several that can be achieved. As noted, a therapeutically effect amount includes amounts that provide a treatment in which the onset or progression of the disorder is delayed, hindered, or prevented, or the disorder or a symptom of the disorder is ameliorated. One or more of the symptoms may be less severe. Recovery may be accelerated in an individual who has been treated.

Amounts effective for this use may depend on the severity of the CNS cancer or neurological disorder and the weight and general state of the subject, but generally range from about 0.05 µg to about 1000 µg *(e.g.,* 0.5-100 µg) of an equivalent amount of the protein conjugate per dose per subject. Suitable regimes for initial administration and booster administrations are typified by an initial administration followed by repeated doses at one or more hourly, daily, weekly, or monthly intervals by a subsequent administration. For example, a subject may receive a protein conjugate in the range of about 0.05 to 1,000 µg equivalent dose as compared to an unconjugated antibody moiety per dose one or more times per week *(e.g.,* 2, 3, 4, 5, 6, or 7 or more times per week). For example, a subject may receive 0.1 to 2,500 µg *(e.g.,* 2,000, 1,500, 1,000, 500, 100, 10, 1, 0.5, or 0.1 µg) dose per week. A subject may also receive a conjugate of the invention in the range of 0.1 to 3,000 µg per dose once every two or three weeks. A subject may also receive 2 mg/kg every week (with the weight calculated based on the weight of the conjugate or the antibody moiety).

The total effective amount of a protein conjugate in the pharmaceutical compositions of the invention can be administered to a mammal as a single dose, either as a bolus or by infusion over a relatively short period of time, or can be administered using a fractionated treatment protocol in which multiple doses are administered over a more prolonged period of time *(e.g.,* a dose every 4-6, 8-12, 14-16, or 18-24 hours, or every 2-4 days, 1-2 weeks, or once a month). Alternatively, continuous intravenous infusions sufficient to maintain therapeutically effective concentrations in the blood are contemplated.

The therapeutically effective amount of one or more agents present within the compositions of the invention and used in the methods of this invention applied to mammals (*e.g.,* humans) can be determined by one of ordinary skill in the art with consideration of individual differences in age, weight, and other general conditions (as mentioned above). Because the protein conjugates of the invention exhibit an enhanced ability to cross the BBB, the dosage of the antibody moiety can be lower than an effective dose of the antibody moiety when unconjugated. For example, the dosage of the antibody moiety can be less than or about 90%, 75%, 50%, 40%, 30%, 20%, 15%, 12%, 10%, 8%, 7%, 6%, 5%, 4%, 3%, 2%, 1%, 0.5%, or 0.1 % of the dose of required for a therapeutic effect with the same or a comparable unconjugated agent.

Therapeutically effective amounts can also be determined empirically by those of skill in the art. For example, either single or multiple administrations of the pharmaceutical compositions of the invention can be carried out with dosage levels and the timing or pattern of administration being selected by the treating physician. The dose and administration schedule can be determined and adjusted based on the severity of the disease or condition in the subject, which may be monitored throughout the course of treatment according to the methods commonly practiced by clinicians or other skilled healthcare professionals.

*Kits:* The kits of the invention can include any combination of the compositions described above and suitable instructions (whether written and/or provided as audio-, visual-, or audiovisual material). In one embodiment, the kit includes a pharmaceutical composition or a precursor thereto that is packaged together with instructions for use and, optionally, any device useful in manipulating the compositions in preparation for administration and/or in administering the compositions. For example, the kits of the invention can include one or more of: diluents, gloves, vials or other containers, pipettes, needles, syringes, tubing, stands, spatulas, sterile cloths or drapes, positive and/or negative controls, and the like. In another embodiment, the kits of the invention include compositions and reagents useful in making a protein conjugate. For example, the kits can include one or more of: an antibody moiety, a linker, a linker-bound antibody moiety, a polypeptide, a chemical entity reactive with the linker, and/or a modified polypeptide. For example, in one embodiment, a kit can include an antibody moiety, a linker, a chemical entity reactive with the linker, and a polypeptide. In other embodiment, the kit can include a linker-bound antibody moiety and a modified polypeptide. As with the kits useful in administering the compositions, kits useful in making the compositions can include any one or more of: diluents, gloves, vials or other containers, pipettes, needles, syringes, tubing, stands, spatulas, sterile cloths or drapes, positive and/or negative controls, and the like. Any reagents useful in binding a linker to an antibody moiety or polypeptide, modifying a polypeptide, conjugating a linker-bound antibody moiety to a modified polypeptide (or vice versa; conjugating an antibody moiety to a linker-bound polypeptide), or purifying and testing a protein conjugate can also be included. As noted, the linker-bound antibody moieties and modified polypeptides are featured compositions of the invention.

### EXAMPLES

### Example 1: Preliminary Study with Varied Conjugation Strategies (H1, H2, and H3)

In a series of experiments aimed at optimizing the conditions for preparing a protein conjugate, we reacted trastuzumab (as the antibody moiety) with the polypeptide represented by SEQ ID NO:97 (Angiopep-2 (An2)). We used two different conjugation chemistries, one of which employed the linker SATA. In this conjugation, the succinimidyl ester within the linker SATA chemically reacts with a primary amine in the polypeptide. Generally, with copper-free click chemistry, two approaches can be used. In one, the antibody moiety is modified with azido groups on the primary amines whereas the polypeptide is modified with alkyne. In the second, the antibody moiety is modified with an alkyne (*e.g.,* MFCO or BCN) whereas the polypeptide is modified with azido groups on the primary amines. We also varied the ratio of the antibody moiety to the polypeptide, from 1:8 to 1:32. The reactions were run at a theoretical 5 mg scale.

| | Chemistry | Equivalents of Angiopep-2 | Angiopep-2 incorporated | % yield based on mAb |
|---|---|---|---|---|
| H1 | SATA | 8 | <1 eq/mAb | 6% |
| H2 | Click, C-terminal of An2 | 32 | <1 eq/mAb | 6% |
| H3 | Click, Lys of An2 | 32 | <1 eq/mAb | 6% |

Because less than 1 equivalent of Angiopep-2 was incorporated, there was a large excess of trastuzumab, and we used an anti-Angiopep-2 affinity column for further purification and removal of trastuzumab. This led to poor yields (between 5-10%). There was also an issue with the traditional click chemistry using a copper catalyst, which remained present in trace amounts and could contribute to instability of the conjugates.

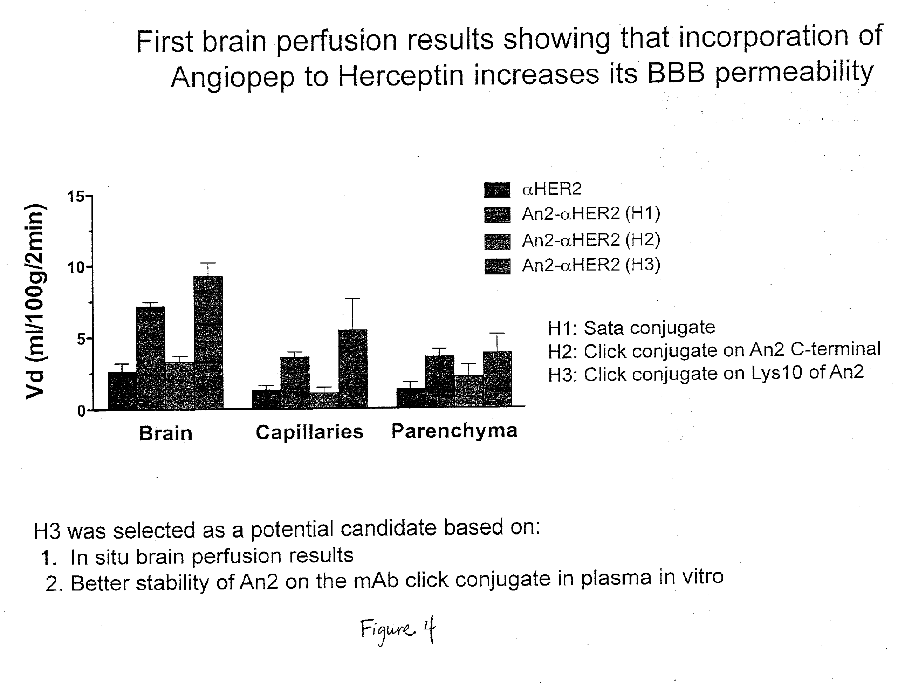

We tested H1, H2, and H3 *in situ* in a study designed to test brain perfusion. The results showed higher levels of H1 and H3 in the brain than trastuzumab (αHER2) alone (Figure 4).

### Example 2: Production and Testing of a Second Series of Conjugates Using Optimized Copper-free Click Chemistry

Copper-free click chemistry technique was used because it is a fast, simple, and reproducible technique that does not require a highly toxic catalyst. Usually, the reaction between alkyne and azide moieties takes place within 24 hours at room temperature and, as noted, does not require the catalyst Cu(I). The reaction conditions are mild and therefore do not cause protein degeneration, and the reactive moieties do not interact with functional groups on biomolecules. Further, the location of the conjugation site on the polypeptide moiety can be controlled and the resulting conjugates are stable and biologically active.

In our production methods, we have designated "step 1" as "activation, purification and dialysis." This step produces a linker-bound antibody moiety. In one experiment, to carry out step 1, we added 200 µl of a stock solution of MFCO-N-hydroxysuccinimide ester to 51 mg of an anti-HER2 monoclonal antibody. In the stock solution of the linker, 7.6 mg of MFCO-N-hydroxysuccinimide ester was dissolved in 1000 µl of anhydrous DMSO. The reaction was left at room temperature for five hours with occasional shaking. The linker-bound antibody moiety was purified from the excess reagent by gel filtration with a desalting column. 35 mL was collected from the column, with the linker-bound antibody moiety being present at a concentration of 1.3 mg/mL. The ligand-bound antibody moiety was dialyzed against phosphate/citrate buffer (pH∼5) overnight at 4°C. 45 mg (yield 88%) of the activated octyne was recovered, which was used for step 2 with azido An2. The formation of the linker-bound antibody moiety (an antibody moiety-octyne) was confirmed by MALDI-TOF analysis.

In step 2, to the linker-bound antibody moiety, we added both an unmodified polypeptide (An2) and a modified polypeptide (An2 linked to a chemical entity comprising N₃). We used 42.8 mg of the linker-bound antibody moiety, 2,156 µl of a stock solution of the unmodified polypeptide (8 equivalents; 13 mg of An2 was dissolved in 4,060 µl of H₂O), and 901 µl of a stock solution of modified polypeptide (10 mg of An2N₃ was dissolved in 1,230 µl of anhydrous DMSO). The mixture was agitated by vortexing, wrapped in aluminum foil, and left overnight at room temperature. The conjugation was monitored by consumption of An2N₃ (the modified polypeptide) by LC-MS. As the concentration of An2 does not change over time, that polypeptide served as an internal standard. The resulting protein conjugate was purified with a Protein A column using a phosphate/citrate buffer at pH 5.0 as the binding buffer and a citric acid buffer at pH 3.0 as the elution buffer. The protein conjugate was isolated and further neutralized with dibasic phosphate buffer to pH 5.0. The solution was centrifuged and decanted to obtain pure protein conjugate. An2 incorporation was confirmed by MALDI-TOF analysis.

We generated five protein conjugates, designated H40-H44 that include trastuzumab as the antibody moiety and the polypeptide represented by SEQ ID NO:97 (Angiopep-2 or An2). The conjugates were generated at 5 mg scale, and the ratio of the antibody moiety to the polypeptide and the lengths of the linkers were varied.

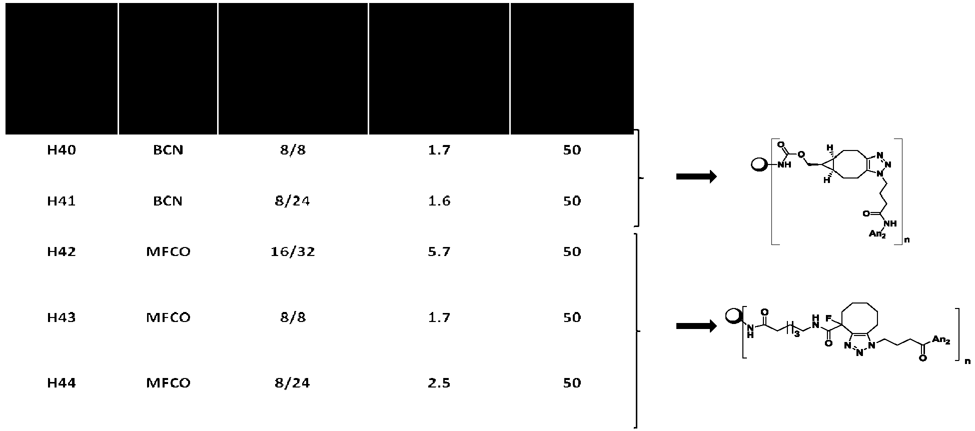

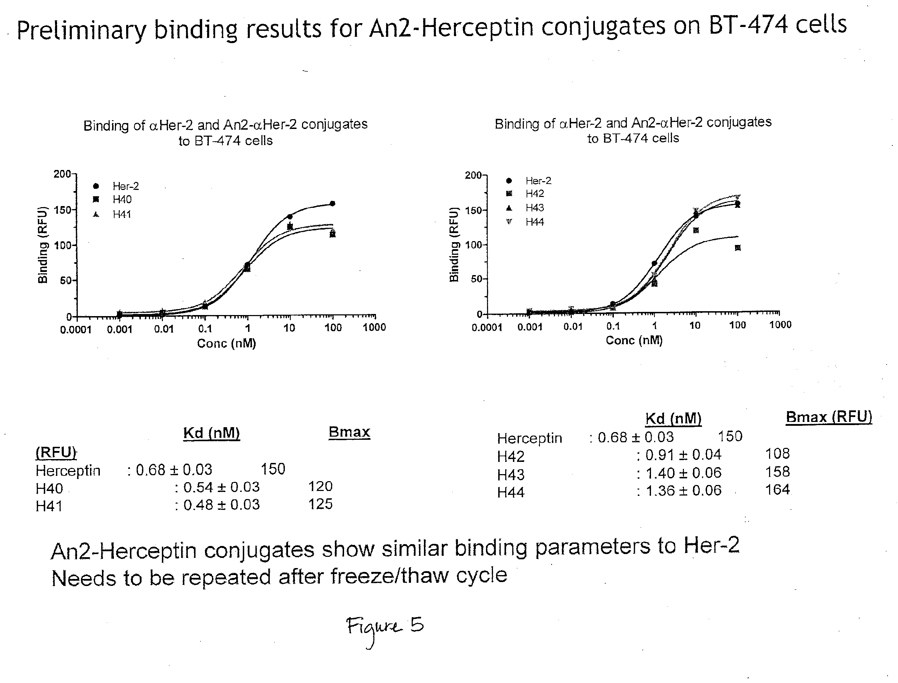

As shown in Figure 5, the protein conjugates H40, H41, H42, H43, and H44 all showed similar binding parameters to trastuzumab. Binding of trastuzumab and An2-trastuzumab conjugates to BT-474 cells was carried out as follows. Confluent BT-474 cells were first detached from flasks with PBS-citrate non-enzymatic dissociating buffer. Cells in suspension were washed in ice-cold binding buffer (BB: Hepes 10 mM, NaCl 150 mM, CaCl₂ 2.5 mM, pH: 7.3), counted and separated in different eppendorfs (10⁶ BT-474 cells per tube). Binding of trastuzumab and An2-trastuzumab conjugates were performed with increasing concentrations of products in ice-cold BB for 30 minutes at 4°C. Cells were then washed and incubated with an α-Human-AlexaFluor488 secondary antibody in ice-cold BB for 30 minutes at 4°C. Cells were extensively washed with ice-cold BB and analyzed by flow cytometry (10,000 gated events per condition).

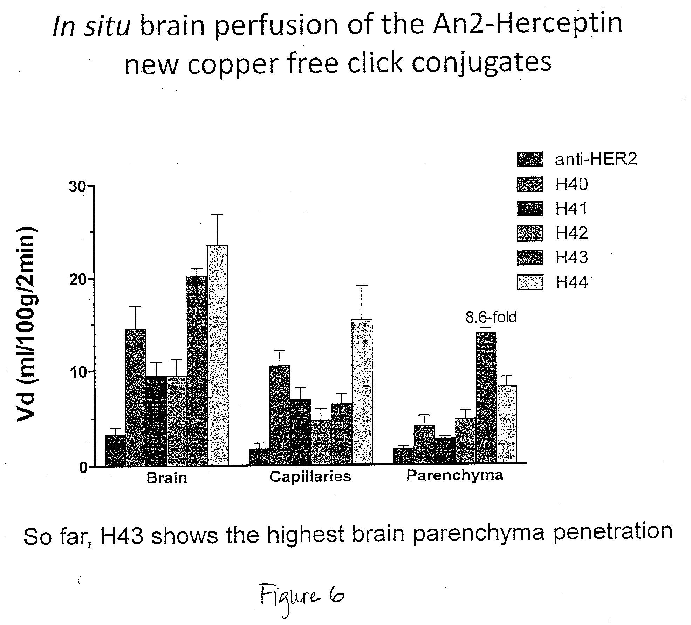

*In situ* brain perfusion studies, carried out as described above for H1, H2, and H3, showed that of the five protein conjugates generated by copper-free click chemistry, H43 showed the highest brain parenchyma penetration (Figure 6). We also observed that H43 was taken up linearly over time between the time of infusion (time 0) and four hours later.

In the Table below, we summarize various characteristics of protein conjugates (H43), comparing first and second batches.

| Conjugate | Activ:An2 | An2 incorporated | Mass | Concentration | Yield |
|---|---|---|---|---|---|
| H43, batch 1 | 8:8 | 1.7 | 152533 | 0.5 mg/ml | 50% |
| H43, batch 2 | 8:8 | 3.6 | 157872 | 1.7 mg/ml | 78% |

The number of An2 polypeptides consumed was determined by LC-MS (liquid chromatography-mass spectrometry). The number of An2 polypeptides incorporated and the mass of the conjugates was determined by MALDI-TOF. The mass of trastuzumab is ∼148,000.

Our work with H43 to date indicates increased brain parenchyma penetration and that the antibody moiety retains the ability to bind Her2 (the binding affinity for Her2 was the same after one freeze/thaw cycle). The composition we produced can be filtered through a 0.2 µm filter without any reduction of the conjugate concentration. The protein conjugate also appears to be stable. After storage at 4°C for fourteen days, no precipitation was observed, the concentration of the conjugate remained largely unchanged after centrifugation and the polypeptide (An2) could still be detected by Western blot after 10 days.

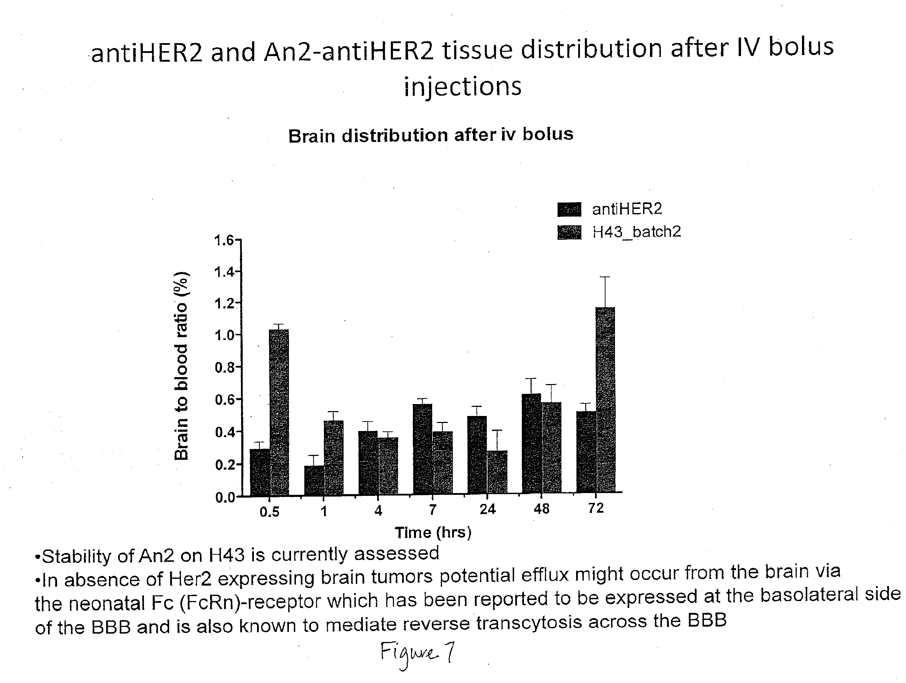

To test tissue distribution, we injected CD-1 mice intravenously with either radiolabeled trastuzumab (¹²⁵I-trastuzumab) or ¹²⁵I-H43 at an equivalent dose of 10 mg/kg. Blood samples were collected 30 minutes, 1 hour, 4 hours, 7 hours, 24 hours, 48 hours, and 72 hours later. The mice were then sacrificed and perfused with cold phosphate-buffered saline for ∼ 8 minutes. Various tissues were harvested and radioactivity was measured to assess tissue distribution. The results are shown in Figure 7. We found that, after 30 minutes, the distribution of H43 in the brain was 3.5-fold greater than that of trastuzumab; after 60 minutes, the distribution was 2.5-fold greater; and after 72 hours, the distribution was 2.3-fold higher. In the absence of Her2-expressing brain tumors, a potential efflux might occur from the brain via the neonatal Fc (FcRn) receptor, which has been reported to be expressed at the basolateral side of the BBB and is also known to mediate reverse transcytosis across the BBB. While the distribution of the protein conjugate was higher in the brain than the unconjugated antibody, the distribution of both the protein conjugate and the unconjugated antibody was lower in the brain than in any other tissue tested (including muscle, heart, liver, kidney, and lung).

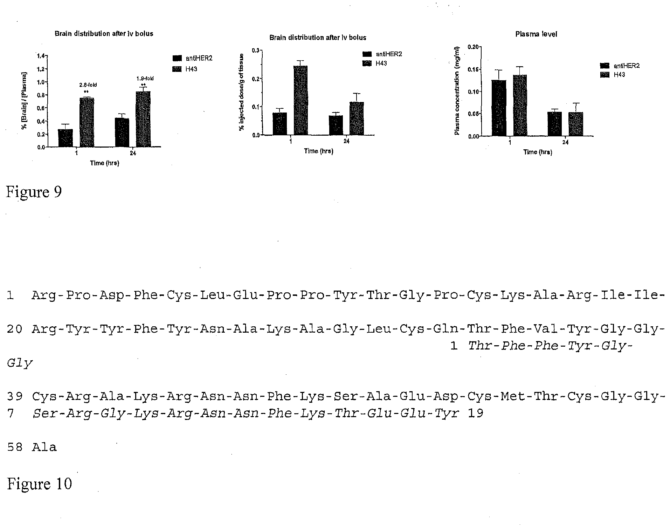

In Figure 9, we present additional data collected from mice treated as described above. The distribution of an unconjugated anti-HER2 antibody (¹²⁵I-trastuzumab) and of the conjugated construct H43 (¹²⁵I-H43) is expressed as % [Brain]/[Plasma] (see the left-hand graph) and as % injected dose/g of tissue (see the center graph). Plasma levels are shown in the right-hand graph.

### Example 3: An Orthotopic Brain Tumor Study in Mice

To test the ability of a protein conjugate to inhibit a brain tumor, BT-474 cells were injected into the brains of 18 mice (that injection defining Day 0). To test the ability of a protein conjugate to inhibit a brain tumor, BT-474 cells were injected into the brains of 18 mice (that injection defining Day 1). On Day 12, the mice were treated with either a control solution (a vehicle-only solution provided by intravenous injection), 14 mg/kg of trastuzumab, or 15 mg/kg of H43. On Day 33, two mice treated according to each treatment regime (two control; 2 trastuzumab; 2 protein conjugate) were sacrificed. Brain tissue was dissected and stained with Trypan blue. No tumors were evident at this time point. On Day 47, two additional mice treated according to each treatment regime were sacrificed. At this time, the mice in the control group had lost weight and appeared to be in poor health. Upon examination, large brain tumors were visible. Visibly smaller tumors were present in the brains of the mice treated with trastuzumab, and small or no tumors were visible in the brains of the mice treated with the protein conjugate. In the mice remaining, treatment continued, followed by intravenous administration of Cyto720 H43 to all mice. For near infrared (NiR) *in vivo* imaging studies, ANG4043 (H43) and anti-Her2 antibody were labeled with a NiR reactive dye Cyto750-NHS ester (Cytodiagnostics, Burlington, ON) according to manufacturer protocol. Briefly, cyto750-NHS ester dissolved in DMSO was incubated with H43 or Herceptin at pH=9 with a dye to protein molar ratio of 1:2. After 1 hour incubation at room temperature, the Cyto750 labeled proteins were separated from the unreactive dye by gel filtration (Pierce Dextran desalting column), the first running colored band being the Cyto750 labelled proteins. Mice were injected with 5 mg/kg of either cyto750-anti-Her2 antibody or cyto750-H43. Animal imaging was performed 24 hrs after injections using the *in vivo* Xtreme imaging system from Carestream.

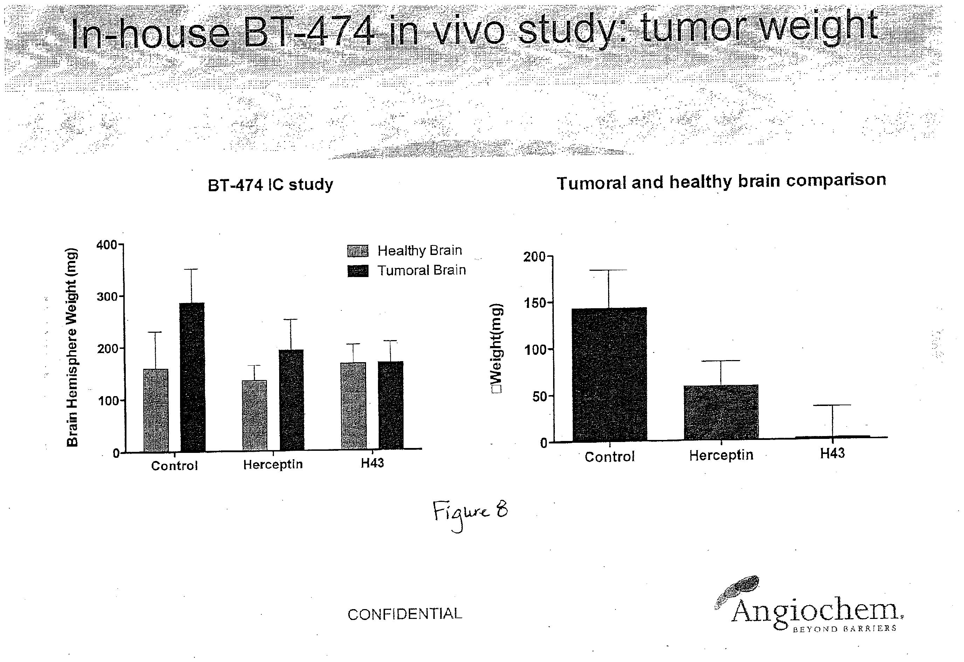

The following day (Day 48, two additional mice treated according to each treatment regime were sacrificed. The results were similar to those reported on Day 47. The weight of the brain was measured as an indication of tumor load, and the results are shown in Figure 8. While the brains of the mice treated with the control solution and the brains of the mice treated with trastuzumab were heavier following BT-474 cell injection than the brains of healthy mice, the brains of mice treated with the protein conjugate following BT-474 cell injection were no heavier than the brains of healthy mice.

### Example 4: Effect of ANG4043 treatment on BT-474 intracerebral tumor progression; an NiR imaging study.

The near infrared lipophilic fluorescent probe DiR was incorporated into BT-474 breast tumor cell membranes. This probe is suitable for inclusion in any of the present conjugates. The BT-474-DiR tumor cells were then implanted in the brains of nude mice. Twelve days after implantation, the mice were treated with vehicle or ANG4043, which is the same conjugate referred to above as H43 (15 mg/kg, IV, twice a week), and subjected to NiR *in vivo* imaging. Images were taken after two and five treatments to monitor tumor progression. After 5 treatments, the tumor-associated fluorescence was greatly decreased in the ANG4043 treated group while tumor-associated fluorescence remained highly detectable in the control group. These results indicate the potential effect of ANG4043 treatment on tumor growth.
In related studies, we found that conjugating an anti-HER2 antibody to an angiopep polypeptide (An2) does not affect the binding affinity of the antibody to BT-474 breast tumor cell membranes. We also further examined the proliferation of BT-474 cells treated with ANG4043 or the unconjugated anti-HER2 antibody (5-day treatment followed by a 4-hour exposure to tritiated thymidine). Incorporation was comparable at lower concentrations (0.0001-0.1 nM), but the ANG4043-treated cells proliferated less at 100 nM. The IC50 (nM) was 4.99 ± 0.76 for the anti-HER2 antibody (n=2) and 3.16 ± 0.97 for the protein conjugate (n=3).

Preliminary PK parameters for ANG4043 (WinNonlin non-compartmental analysis) are shown in the following Table:

| Parameter | anti-HER2 antibody | ANG4043 |
|---|---|---|
| Cmax (µg/ml) | 166 | 91.5 |
| T1/2β (day) | 11.4 | 8.5 |
| AUC0-30 (day*µg/ml) | 573.3 | 275.4 |
| AUC0-∞ (day*µg/ml) | 703.6 | 310.3 |
| CI (ml/day) | 0.43 | 0.97 |
| Vd (ml) | 7.3 | 11.8 |
| MRT (day) | 16.7 | 12.2 |

In *in vivo* studies in rodents, we found a reduction in HER2-positive tumor size with ANG4043. BT-474 tumor cells were pre-loaded with a fluorescent dye, DiR, prior to intracranial implantation in mice (day 1). Mice were treated twice weekly with anti-HER2 and ANG4043, 20 mg/kg, beginning on day 12. NiR imaging was performed on day 16, at which point two treatment had been administered, and on day 33, after seven treatments. By fluorescence imaging, we found that ANG4043 co-localizes with cell-surface HER2 in BT-474 cells and is internalized to a greater extent than Herceptin. ANG4043-treated brains had less tumor growth (as observed, for example, by NiR imaging). In terms of survival:

| Group | Mean Survival (Days) |
|---|---|
| Control | 47 |
| Herceptin (5 mg/kg) | 52 + 11% (p=0.028) |
| ANG4043 (5 mg/kg) | 60 + 28% (p=0.0012) |

## Claims

1. A protein conjugate comprising an antibody moiety, a polypeptide comprising the amino acid sequence Thr₁-Phe₂-Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO: 117), or a biologically active analog or fragment thereof, and a linker between the antibody moiety and the polypeptide,
wherein the antibody moiety is trastuzumab, cetuximab, or panitumumab, or a biologically active variant thereof,
wherein the antibody moiety is conjugated to the polypeptide at one or more of the following positions on the polypeptide: the N-terminal amino acid residue; the C-terminal amino acid residue; or a lysine residue between the N-terminus and the C-terminus, and wherein the linker is a monofluoro cyclooctyne (MFCO), bicyclo[6.1.0]nonyne (BCN), or dibenzocyclooctyne (DBCO).

2. The conjugate of claim 1,
wherein the polypeptide comprises the amino acid sequence Thr₁-Phe₂-Phe₃-Tyr₄-Gly₅-Gl_{Y6}-Cys₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO:67), or
wherein the polypeptide comprises the amino acid sequence Thr₁-Phe₂-Phe₃-Tyr₄-Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO: 97).

3. The conjugate of claim 1, wherein the conjugate comprises an analog of SEQ ID NO: 117,
preferably
wherein the analog of SEQ ID NO: 117 comprises at least 13 amino acid residues that are invariant relative to SEQ ID NO: 117, or
wherein the analog of SEQ ID NO: 117 comprises a sequence in which Lys₁₀ and/or Lys₁₅ remain invariant, or
wherein, in the analog of SEQ ID NO: 117, Asn₁₂ is substituted with Gln, Asn₁₃ is substituted with Gln, and/or Phe₁₄ is substituted with Tyr or Trp, or
wherein the analog of SEQ ID NO: 117 comprises the sequence Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ IDNO: 118); Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO:119); Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 120); Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 121); or Lys₁₀-Arg₁₁-Asn₁₂-Asn₁₃-Phe₁₄-Lys₁₅-Tyr₁₉-Cys (SEQ ID NO: 122).

4. The conjugate of claim 1, wherein the polypeptide comprises a fragment of SEQ ID NO:117.

5. The conjugate of claim 4, wherein the fragment comprises at least six amino acid residues of SEQ ID NO:117,
preferably wherein the fragment is Lys₁₀-Arg₁₁-Asn₁₂-Asn₁₃-Phe₁₄-Lys₁₅ (SEQ ID NO: 123).

6. The conjugate of claim 4, wherein the fragment comprises 6-18 contiguous amino acid residues of SEQ ID NO: 117.

7. The conjugate of claim 1,
(a) wherein the polypeptide comprises amino acid residues in the D-form, the L-form, or both forms, or
(b) wherein the conjugate comprises 1-10 polypeptides and one antibody moiety, preferably wherein the conjugate comprises 2-6 polypeptides and one antibody moiety, or
(c) wherein each polypeptide is linked, via a linker, to an antibody moiety, and no polypeptide is linked, via the linker or otherwise, to another polypeptide, or
(d) wherein the antibody moiety comprises a human, chimeric or humanized antibody or a biologically active variant thereof, or
(e) wherein the antibody moiety is a monoclonal antibody or a polyclonal antibody, or
(f) wherein the antibody moiety is a chemotherapeutic agent, or
(g) wherein the antibody moiety is an anti-inflammatory agent.

8. The conjugate of claim 1,
(a) wherein the antibody moiety is a tetrameric antibody or a biologically active variant thereof, or
(b) wherein the antibody moiety is a single chain antibody (scFv), Fab fragment, or F(ab')₂ fragment.

9. A pharmaceutical composition comprising the conjugate of claim 1 and a pharmaceutically acceptable carrier, preferably wherein the composition is formulated for intravenous administration.

10. The pharmaceutical composition of claim 9 for use in a method of treating a patient who is suffering from a cancer of the central nervous system, wherein the method comprises:
identifying a patient in need of treatment; and
administering to the patient a therapeutically effective amount of the pharmaceutical composition.

11. The pharmaceutical composition for use of claim 10, wherein the patient is a human patient, or wherein the cancer is a brain tumor or a brain metastasis.

## Patentansprüche

1. Proteinkonjugat, umfassend eine Antikörpergruppierung, ein Polypeptid umfassend die Aminosäuresequenz Thr₁-Phe₂-Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO: 117), oder ein biologisch aktives Analogon oder Fragment davon, und einen Linker zwischen der Antikörpergruppierung und dem Polypeptid,
wobei die Antikörpergruppierung Trastuzumab, Cetuximab oder Panitumumab oder eine biologisch aktive Variante davon ist,
wobei die Antikörpergruppierung mit dem Polypeptid an einer oder mehreren der folgenden Positionen auf dem Polypeptid konjugiert ist: dem N-terminalen Aminosäurerest; dem C-terminalen Aminosäurerest; oder einem Lysinrest zwischen dem N-Terminus und dem C-Terminus, und wobei der Linker ein Monofluorcyclooctin (MFCO), Bicyclo[6.1.0]nonin (BCN) oder Dibenzocyclooctin (DBCO) ist.

2. Konjugat nach Anspruch 1,
wobei das Polypeptid die Aminosäuresequenz Thr₁-Phe₂-Phe₃-Tyr₄-Gly₅-Gly₆-Cys₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO: 67) umfasst, oder
wobei das Polypeptid die Aminosäuresequenz Thr₁-Phe₂-Phe₃-Tyr₄-Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO: 97) umfasst.

3. Konjugat nach Anspruch 1, wobei das Konjugat ein Analogon von SEQ ID NO: 117 umfasst,
vorzugsweise
wobei das Analogon von SEQ ID NO: 117 mindestens 13 Aminosäurereste umfasst, die relativ zu SEQ ID NO: 117 invariant sind, oder
wobei das Analogon von SEQ ID NO: 117 eine Sequenz umfasst, bei welcher Lys₁₀ und/oder Lysis invariant bleiben/bleibt, oder
wobei in dem Analogon von SEQ ID NO: 117 Asn₁₂ mit Gln substituiert ist, Asn₁₃ mit Gln substituiert ist, und/oder Phe₁₄ mit Tyr oder Trp substituiert ist, oder
wobei das Analogon von SEQ ID NO: 117 die Sequenz Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 118); Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 119); Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 120); Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO: 121); oder Lys₁₀-Arg₁₁-Asn₁₂-Asn₁₃-Phe₁₄-Lys₁₅-Tyr₁₉-Cys (SEQ ID NO: 122) umfasst.

4. Konjugat nach Anspruch 1, wobei das Polypeptid ein Fragment von SEQ ID NO: 117 umfasst.

5. Konjugat nach Anspruch 4, wobei das Fragment mindestens sechs Aminosäurereste von SEQ ID NO: 117 umfasst,
vorzugsweise wobei das Fragment Lys₁₀-Arg₁₁-Asn₁₂-Asn₁₃-Phe₁₄-Lys₁₅ (SEQ ID NO: 123) ist.

6. Konjugat nach Anspruch 4, wobei das Fragment 6-18 zusammenhängende Aminosäurereste von SEQ ID NO: 117 umfasst.

7. Konjugat nach Anspruch 1,
(a) wobei das Polypeptid Aminosäurereste in der D-Form, der L-Form oder beiden Formen umfasst, oder
(b) wobei das Konjugat 1-10 Polypeptide und eine Antikörpergruppierung umfasst, vorzugsweise wobei das Konjugat 2-6 Polypeptide und eine Antikörpergruppierung umfasst, oder
(c) wobei jedes Polypeptid über einen Linker mit einer Antikörpergruppierung verknüpft ist, und kein Polypeptid über den Linker oder auf andere Weise mit einem anderen Polypeptid verknüpft ist, oder
(d) wobei die Antikörpergruppierung einen humanen, chimären oder humanisierten Antikörper oder eine biologisch aktive Variante davon umfasst, oder
(e) wobei die Antikörpergruppierung ein monoklonaler Antikörper oder ein polyklonaler Antikörper ist, oder
(f) wobei die Antikörpergruppierung ein chemotherapeutisches Mittel ist, oder
(g) wobei die Antikörpergruppierung ein entzündungshemmendes Mittel ist.

8. Konjugat nach Anspruch 1,
(a) wobei die Antikörpergruppierung ein tetramerer Antikörper oder eine biologisch aktive Variante davon ist, oder
(b) wobei die Antikörpergruppierung ein Einzelkettenantikörper (scFv), Fab-Fragment, oder F(ab')₂-Fragment ist.

9. Pharmazeutische Zusammensetzung, umfassend das Konjugat gemäß Anspruch 1 und einen pharmazeutisch annehmbaren Träger, vorzugsweise wobei die Zusammensetzung zur intravenösen Verabreichung formuliert ist.

10. Pharmazeutische Zusammensetzung gemäß Anspruch 9 zur Verwendung in einem Verfahren zur Behandlung eines Patienten, der an einem Krebs des Zentralnervensystems leidet, wobei das Verfahren umfasst:
Identifizieren eines Patienten, der einer Behandlung bedarf; und
Verabreichen einer therapeutisch wirksamen Menge der pharmazeutischen Zusammensetzung an den Patienten.

11. Pharmazeutische Zusammensetzung zur Verwendung nach Anspruch 10, wobei der Patient ein humaner Patient ist, oder wobei der Krebs ein Gehirntumor oder eine Gehirnmetastase ist.

## Revendications

1. Conjugué protéique comprenant un groupement anticorps, un polypeptide comprenant la séquence d'acides aminés Thr₁-Phe₂-Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO : 117), ou un analogue ou groupement biologiquement actif de celui-ci, et un lieur entre le groupement anticorps et le polypeptide,
dans lequel le groupement anticorps est le trastuzumab, le cétuximab, ou le panitumumab, ou un variant biologiquement actif de celui-ci,
dans lequel le groupement anticorps est conjugué au polypeptide au niveau d'une ou plusieurs des positions suivantes sur le polypeptide : le résidu d'acide aminé N-terminal ; le résidu d'acide aminé C-terminal ; ou un résidu de lysine entre l'extrémité N et l'extrémité C, et dans lequel le lieur est un monofluoro-cyclooctyne (MFCO), bicyclo[6.1.0]nonyne (BCN) ou dibenzocyclooctyne (DBCO).

2. Conjugué selon la revendication 1,
dans lequel le polypeptide comprend la séquence d'acides aminés Thr₁-Phe₂-Phe₃-Tyr₄-Gly₅-Gly₆-Cys₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO : 67), ou
dans lequel le polypeptide comprend la séquence d'acides aminés Thr₁-Phe₂-Phe₃-Tyr₄-Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉ (SEQ ID NO : 97).

3. Conjugué selon la revendication 1, où le conjugué comprend un analogue de la SEQ ID NO : 117,
de préférence
dans lequel l'analogue de la SEQ ID NO : 117 comprend au moins 13 résidus d'acide aminé qui sont invariants par rapport à la SEQ ID NO : 117, ou
dans lequel l'analogue de la SEQ ID NO : 117 comprend une séquence dans laquelle Lys₁₀ et/ou Lys₁₅ restent invariants, ou
dans lequel, dans l'analogue de la SEQ ID NO : 117, Asn₁₂ est remplacé par Gln, Asn₁₃ est remplacé par Gln, et/ou Phe₁₄ est remplacé par Tyr ou Trp, ou
dans lequel l'analogue de la SEQ ID NO : 117 comprend la séquence Phe₃-Tyr₄-Gly₅-Gly₆-Cys₇/Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO : 118) ; Gly₅-Gly₆-Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO : 119) ; Ser₇-Arg₈-Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO : 120) ; Gly₉-Lys₁₀-Arg₁₁-Asn₁₂-Asn₁₃-Phe₁₄-Lys₁₅-Thr₁₆-Glu₁₇-Glu₁₈-Tyr₁₉-Cys (SEQ ID NO : 121) ; ou Lys₁₀-Arg₁₁-Asn₁₂-Asn₁₃-Phe₁₄-Lys₁₅-Tyr₁₉-Cys (SEQ ID NO : 122).

4. Conjugué selon la revendication 1, dans lequel le polypeptide comprend un groupement de la SEQ ID NO : 117.

5. Conjugué selon la revendication 4, dans lequel le groupement comprend au moins six résidus d'acide aminé de la SEQ ID NO : 117,
de préférence dans lequel le groupement est Lys₁₀-Arg₁₁-Asn₁₂-Asn₁₃-Phe₁₄-Lys₁₅ (SEQ ID NO : 123) .

6. Conjugué selon la revendication 4, dans lequel le groupement comprend 6 à 18 résidus d'acide aminé contigus de la SEQ ID NO : 117.

7. Conjugué selon la revendication 1,
(a) dans lequel le polypeptide comprend des résidus d'acide aminé sous forme D, sous forme L, ou sous les deux formes, ou
(b) où le conjugué comprend 1 à 10 polypeptides et un seul groupement anticorps, de préférence où le conjugué comprend 2 à 6 polypeptides et un seul groupement anticorps, ou
(c) dans lequel chaque polypeptide est lié, via un lieur, à un groupement anticorps, et aucun polypeptide n'est lié, via le lieur ou autrement, à un autre polypeptide, ou
(d) dans lequel le groupement anticorps comprend un anticorps humain, chimère ou humanisé ou un variant biologiquement actif de celui-ci, ou
(e) dans lequel le groupement anticorps est un anticorps monoclonal ou un anticorps polyclonal, ou
(f) dans lequel le groupement anticorps est un agent chimiothérapeutique, ou
(g) dans lequel le groupement anticorps est un agent anti-inflammatoire.

8. Conjugué selon la revendication 1,
(a) dans lequel le groupement anticorps est un anticorps tétramère ou un variant biologiquement actif de celui-ci, ou
(b) dans lequel le groupement anticorps est un anticorps à une seule chaîne (scFv), un fragment Fab, ou un fragment F(ab')₂.

9. Composition pharmaceutique comprenant le conjugué de la revendication 1 et un véhicule pharmaceutiquement acceptable, de préférence où la composition est formulée pour une administration par voie intraveineuse.

10. Composition pharmaceutique selon la revendication 9 pour utilisation dans une méthode de traitement d'un patient qui souffre d'un cancer du système nerveux central, où la méthode comprend :
l'identification d'un patient ayant besoin du traitement ; et
l'administration au patient d'une quantité efficace du point de vue thérapeutique de la composition pharmaceutique.

11. Composition pharmaceutique pour utilisation selon la revendication 10, où le patient est un patient humain, ou où le cancer est une tumeur cérébrale ou une métastase cérébrale.


### SEQUENCE LISTING

<110> ANGIOCHEM INC.
<120> APROTININ-DERIVED POLYPEPTIDE-ANTIBODY CONJUGATES
<130> G69729PCEP
<140> EP 13853308.8
   <141> 2013-11-12
<150> US 61/725,365
   <151> 2012-11-12
<160> 129
<170> PatentIn version 3.5
<210> 1
   <211> 16
   <212> PRT
   <213> Artificial Sequence
<220>
   <221> source
   <223> /note="Description of Artificial Sequence: Synthetic peptide"
<400> 1
   Thr Phe Val Tyr Gly Gly Cys Arg Ala Lys Arg Asn Asn Phe Lys Ser
   1               5                   10                  15
<210> 2
   <211> 19
   <212> PRT
   <213> Artificial Sequence
<220>
   <221> source
   <223> /note="Description of Artificial Sequence: Synthetic peptide"
<400> 2
   Thr Phe Gln Tyr Gly Gly Cys Met Gly Asn Gly Asn Asn Phe Val Thr
   1               5                   10                  15
   Glu Lys Glu
<210> 3
   <211> 19
   <212> PRT
   <213> Artificial Sequence
<220>
   <221> source
   <223> /note="Description of Artificial Sequence: Synthetic peptide"
<400> 3
   Pro Phe Phe Tyr Gly Gly Cys Gly Gly Asn Arg Asn Asn Phe Asp Thr
   1 5 10 15
   Glu Glu Tyr
<210> 4
   <211> 19
   <212> PRT
   <213> Artificial Sequence
<220>
   <221> source
   <223> /note="Description of Artificial Sequence: Synthetic peptide"
<400> 4
   Ser Phe Tyr Tyr Gly Gly Cys Leu Gly Asn Lys Asn Asn Tyr Leu Arg
   1 5 10 15
   Glu Glu Glu
<210> 5
   <211> 19
   <212> PRT
   <213> Artificial Sequence
<220>
   <221> source
   <223> /note="Description of Artificial Sequence: Synthetic peptide"
<400> 5
   Thr Phe Phe Tyr Gly Gly Cys Arg Ala Lys Arg Asn Asn Phe Lys Arg
   1 5 10 15
   Ala Lys Tyr
<210> 6
   <211> 19
   <212> PRT
   <213> Artificial Sequence
<220>
   <221> source
   <223> /note="Description of Artificial Sequence: Synthetic peptide"
<400> 6
   Thr Phe Phe Tyr Gly Gly Cys Arg Gly Lys Arg Asn Asn Phe Lys Arg
   1 5 10 15
   Ala Lys Tyr
<210> 7
   <211> 19
   <212> PRT
   <213> Artificial Sequence
<220>
   <221> source
   <223> /note="Description of Artificial Sequence: Synthetic peptide"
<400> 7
   Thr Phe Phe Tyr Gly Gly Cys Arg Ala Lys Lys Asn Asn Tyr Lys Arg
   1               5                   10                  15
   Ala Lys Tyr
<210> 8
   <211> 19
   <212> PRT
   <213> Artificial Sequence
<220>
   <221> source
   <223> /note="Description of Artificial Sequence: Synthetic peptide"
<400> 8
   Thr Phe Phe Tyr Gly Gly Cys Arg Gly Lys Lys Asn Asn Phe Lys Arg
   1               5                   10                  15
   Ala Lys Tyr
<210> 9
   <211> 19
   <212> PRT
   <213> Artificial Sequence
<220>
   <221> source
   <223> /note="Description of Artificial Sequence: Synthetic peptide"
<400> 9
   Thr Phe Gln Tyr Gly Gly Cys Arg Ala Lys Arg Asn Asn Phe Lys Arg
   1               5                   10                  15
   Ala Lys Tyr
<210> 10
   <211> 19
   <212> PRT
   <213> Artificial Sequence
<220>
   <221> source
   <223> /note="Description of Artificial Sequence: Synthetic peptide"
<400> 10
   Thr Phe Gln Tyr Gly Gly Cys Arg Gly Lys Lys Asn Asn Phe Lys Arg
   1 5 10 15
   Ala Lys Tyr
<210> 11
   <211> 19
   <212> PRT
   <213> Artificial Sequence
<220>
   <221> source
   <223> /note="Description of Artificial Sequence: Synthetic peptide"
<400> 11
   Thr Phe Phe Tyr Gly Gly Cys Leu Gly Lys Arg Asn Asn Phe Lys Arg
   1 5 10 15
   Ala Lys Tyr
<210> 12
   <211> 19
   <212> PRT
   <213> Artificial Sequence
<220>
   <221> source
   <223> /note="Description of Artificial Sequence: Synthetic peptide"
<400> 12
   Thr Phe Phe Tyr Gly Gly Ser Leu Gly Lys Arg Asn Asn Phe Lys Arg
   1 5 10 15
   Ala Lys Tyr
<210> 13
   <211> 19
   <212> PRT
   <213> Artificial Sequence
<220>
   <221> source
   <223> /note="Description of Artificial Sequence: Synthetic peptide"
<400> 13
   Pro Phe Phe Tyr Gly Gly Cys Gly Gly Lys Lys Asn Asn Phe Lys Arg
   1 5 10 15
   Ala Lys Tyr
<210> 14
   <211> 19
   <212> PRT
   <213> Artificial Sequence
<220>
   <221> source
   <223> /note="Description of Artificial Sequence: Synthetic peptide"
<400> 14
   Thr Phe Phe Tyr Gly Gly Cys Arg Gly Lys Gly Asn Asn Tyr Lys Arg
   1 5 10 15
   Ala Lys Tyr
<210> 15
   <211> 19
   <212> PRT
   <213> Artificial Sequence
<220>
   <221> source
   <223> /note="Description of Artificial Sequence: Synthetic peptide"
<400> 15
   Pro Phe Phe Tyr Gly Gly Cys Arg Gly Lys Arg Asn Asn Phe Leu Arg
   1 5 10 15
   Ala Lys Tyr
<210> 16
   <211> 19
   <212> PRT
   <213> Artificial Sequence
<220>
   <221> source
   <223> /note="Description of Artificial Sequence: Synthetic peptide"
<400> 16
   Thr Phe Phe Tyr Gly Gly Cys Arg Gly Lys Arg Asn Asn Phe Lys Arg
   1 5 10 15
   Glu Lys Tyr
<210> 17
   <211> 19
   <212> PRT
   <213> Artificial Sequence
<220>
   <221> source
   <223> /note="Description of Artificial Sequence: Synthetic peptide"
<400> 17
   Pro Phe Phe Tyr Gly Gly Cys Arg Ala Lys Lys Asn Asn Phe Lys Arg
   1               5                   10                  15
   Ala Lys Glu
<210> 18
   <211> 19
   <212> PRT
   <213> Artificial Sequence
<220>
   <221> source
   <223> /note="Description of Artificial Sequence: Synthetic peptide"
<400> 18
   Thr Phe Phe Tyr Gly Gly Cys Arg Gly Lys Arg Asn Asn Phe Lys Arg
   1               5                   10                  15
   Ala Lys Asp
<210> 19
   <211> 19
   <212> PRT
   <213> Artificial Sequence
<220>
   <221> source
   <223> /note="Description of Artificial Sequence: Synthetic peptide"
<400> 19
   Thr Phe Phe Tyr Gly Gly Cys Arg Ala Lys Arg Asn Asn Phe Asp Arg
   1               5                   10                  15
   Ala Lys Tyr
<210> 20
   <211> 19
   <212> PRT
   <213> Artificial Sequence
<220>
   <221> source
   <223> /note="Description of Artificial Sequence: Synthetic peptide"
<400> 20
   Thr Phe Phe Tyr Gly Gly Cys Arg Gly Lys Lys Asn Asn Phe Lys Arg
   1               5                   10                  15
   Ala Glu Tyr
<210> 21
   <211> 19
   <212> PRT
   <213> Artificial Sequence
<220>
   <221> source
   <223> /note="Description of Artificial Sequence: Synthetic peptide"
<400> 21
   Pro Phe Phe Tyr Gly Gly Cys Gly Ala Asn Arg Asn Asn Phe Lys Arg
   1               5                   10                  15
   Ala Lys Tyr
<210> 22
   <211> 19
   <212> PRT
   <213> Artificial Sequence
<220>
   <221> source
   <223> /note="Description of Artificial Sequence: Synthetic peptide"
<400> 22
   Thr Phe Phe Tyr Gly Gly Cys Gly Gly Lys Lys Asn Asn Phe Lys Thr
   1               5                   10                  15
   Ala Lys Tyr
<210> 23
   <211> 19
   <212> PRT
   <213> Artificial Sequence
<220>
   <221> source
   <223> /note="Description of Artificial Sequence: Synthetic peptide"
<400> 23
   Thr Phe Phe Tyr Gly Gly Cys Arg Gly Asn Arg Asn Asn Phe Leu Arg
   1               5                   10                  15
   Ala Lys Tyr
<210> 24
   <211> 19
   <212> PRT
   <213> Artificial Sequence
<220>
   <221> source
   <223> /note="Description of Artificial Sequence: Synthetic peptide"
<400> 24
   Thr Phe Phe Tyr Gly Gly Cys Arg Gly Asn Arg Asn Asn Phe Lys Thr
   1               5                   10                  15
   Ala Lys Tyr
<210> 25
   <211> 19
   <212> PRT
   <213> Artificial Sequence
<220>
   <221> source
   <223> /note="Description of Artificial Sequence: Synthetic peptide"
<400> 25
   Thr Phe Phe Tyr Gly Gly Ser Arg Gly Asn Arg Asn Asn Phe Lys Thr
   1               5                   10                  15
   Ala Lys Tyr
<210> 26
   <211> 19
   <212> PRT
   <213> Artificial Sequence
<220>
   <221> source
   <223> /note="Description of Artificial Sequence: Synthetic peptide"
<400> 26
   Thr Phe Phe Tyr Gly Gly Cys Leu Gly Asn Gly Asn Asn Phe Lys Arg
   1               5                   10                  15
   Ala Lys Tyr
<210> 27
   <211> 19
   <212> PRT
   <213> Artificial Sequence
<220>
   <221> source
   <223> /note="Description of Artificial Sequence: Synthetic peptide"
<400> 27
   Thr Phe Phe Tyr Gly Gly Cys Leu Gly Asn Arg Asn Asn Phe Leu Arg
   1 5 10 15
   Ala Lys Tyr
<210> 28
   <211> 19
   <212> PRT
   <213> Artificial Sequence
<220>
   <221> source
   <223> /note="Description of Artificial Sequence: Synthetic peptide"
<400> 28
   Thr Phe Phe Tyr Gly Gly Cys Leu Gly Asn Arg Asn Asn Phe Lys Thr
   1 5 10 15
   Ala Lys Tyr
<210> 29
   <211> 19
   <212> PRT
   <213> Artificial Sequence
<220>
   <221> source
   <223> /note="Description of Artificial Sequence: Synthetic peptide"
<400> 29
   Thr Phe Phe Tyr Gly Gly Cys Arg Gly Asn Gly Asn Asn Phe Lys Ser
   1 5 10 15
   Ala Lys Tyr
<210> 30
   <211> 19
   <212> PRT
   <213> Artificial Sequence
<220>
   <221> source
   <223> /note="Description of Artificial Sequence: Synthetic peptide"
<400> 30
   Thr Phe Phe Tyr Gly Gly Cys Arg Gly Lys Lys Asn Asn Phe Asp Arg
   1 5 10 15
   Glu Lys Tyr
<210> 31
   <211> 19
   <212> PRT
   <213> Artificial Sequence
<220>
   <221> source
   <223> /note="Description of Artificial Sequence: Synthetic peptide"
<400> 31
   Thr Phe Phe Tyr Gly Gly Cys Arg Gly Lys Arg Asn Asn Phe Leu Arg
   1               5                   10                  15
   Glu Lys Glu
<210> 32
   <211> 19
   <212> PRT
   <213> Artificial Sequence
<220>
   <221> source
   <223> /note="Description of Artificial Sequence: Synthetic peptide"
<400> 32
   Thr Phe Phe Tyr Gly Gly Cys Arg Gly Lys Gly Asn Asn Phe Asp Arg
   1               5                   10                  15
   Ala Lys Tyr
<210> 33
   <211> 19
   <212> PRT
   <213> Artificial Sequence
<220>
   <221> source
   <223> /note="Description of Artificial Sequence: Synthetic peptide"
<400> 33
   Thr Phe Phe Tyr Gly Gly Ser Arg Gly Lys Gly Asn Asn Phe Asp Arg
   1               5                   10                  15
   Ala Lys Tyr
<210> 34
   <211> 19
   <212> PRT
   <213> Artificial Sequence
<220>
   <221> source
   <223> /note="Description of Artificial Sequence: Synthetic peptide"
<400> 34
   Thr Phe Phe Tyr Gly Gly Cys Arg Gly Asn Gly Asn Asn Phe Val Thr
   1 5 10 15
   Ala Lys Tyr
<210> 35
   <211> 19
   <212> PRT
   <213> Artificial Sequence
<220>
   <221> source
   <223> /note="Description of Artificial Sequence: Synthetic peptide"
<400> 35
   Pro Phe Phe Tyr Gly Gly Cys Gly Gly Lys Gly Asn Asn Tyr Val Thr
   1 5 10 15
   Ala Lys Tyr
<210> 36
   <211> 19
   <212> PRT
   <213> Artificial Sequence
<220>
   <221> source
   <223> /note="Description of Artificial Sequence: Synthetic peptide"
<400> 36
   Thr Phe Phe Tyr Gly Gly Cys Leu Gly Lys Gly Asn Asn Phe Leu Thr
   1 5 10 15
   Ala Lys Tyr
<210> 37
   <211> 19
   <212> PRT
   <213> Artificial Sequence
<220>
   <221> source
   <223> /note="Description of Artificial Sequence: Synthetic peptide"
<400> 37
   Ser Phe Phe Tyr Gly Gly Cys Leu Gly Asn Lys Asn Asn Phe Leu Thr
   1 5 10 15
   Ala Lys Tyr
<210> 38
   <211> 19
   <212> PRT
   <213> Artificial Sequence
<220>
   <221> source
   <223> /note="Description of Artificial Sequence: Synthetic peptide"
<400> 38
   Thr Phe Phe Tyr Gly Gly Cys Gly Gly Asn Lys Asn Asn Phe Val Arg
   1 5 10 15
   Glu Lys Tyr
<210> 39
   <211> 19
   <212> PRT
   <213> Artificial Sequence
<220>
   <221> source
   <223> /note="Description of Artificial Sequence: Synthetic peptide"
<400> 39
   Thr Phe Phe Tyr Gly Gly Cys Met Gly Asn Lys Asn Asn Phe Val Arg
   1 5 10 15
   Glu Lys Tyr
<210> 40
   <211> 19
   <212> PRT
   <213> Artificial Sequence
<220>
   <221> source
   <223> /note="Description of Artificial Sequence: Synthetic peptide"
<400> 40
   Thr Phe Phe Tyr Gly Gly Ser Met Gly Asn Lys Asn Asn Phe Val Arg
   1 5 10 15
   Glu Lys Tyr
<210> 41
   <211> 19
   <212> PRT
   <213> Artificial Sequence
<220>
   <221> source
   <223> /note="Description of Artificial Sequence: Synthetic peptide"
<400> 41
   Pro Phe Phe Tyr Gly Gly Cys Leu Gly Asn Arg Asn Asn Tyr Val Arg
   1               5                   10                  15
   Glu Lys Tyr
<210> 42
   <211> 19
   <212> PRT
   <213> Artificial Sequence
<220>
   <221> source
   <223> /note="Description of Artificial Sequence: Synthetic peptide"
<400> 42
   Thr Phe Phe Tyr Gly Gly Cys Leu Gly Asn Arg Asn Asn Phe Val Arg
   1               5                   10                  15
   Glu Lys Tyr
<210> 43
   <211> 19
   <212> PRT
   <213> Artificial Sequence
<220>
   <221> source
   <223> /note="Description of Artificial Sequence: Synthetic peptide"
<400> 43
   Thr Phe Phe Tyr Gly Gly Cys Leu Gly Asn Lys Asn Asn Tyr Val Arg
   1               5                   10                  15
   Glu Lys Tyr
<210> 44
   <211> 19
   <212> PRT
   <213> Artificial Sequence
<220>
   <221> source
   <223> /note="Description of Artificial Sequence: Synthetic peptide"
<400> 44
   Thr Phe Phe Tyr Gly Gly Cys Gly Gly Asn Gly Asn Asn Phe Leu Thr
   1               5                   10                  15
   Ala Lys Tyr
<210> 45
   <211> 19
   <212> PRT
   <213> Artificial Sequence
<220>
   <221> source
   <223> /note="Description of Artificial Sequence: Synthetic peptide"
<400> 45
   Thr Phe Phe Tyr Gly Gly Cys Arg Gly Asn Arg Asn Asn Phe Leu Thr
   1               5                   10                  15
   Ala Glu Tyr
<210> 46
   <211> 19
   <212> PRT
   <213> Artificial Sequence
<220>
   <221> source
   <223> /note="Description of Artificial Sequence: Synthetic peptide"
<400> 46
   Thr Phe Phe Tyr Gly Gly Cys Arg Gly Asn Gly Asn Asn Phe Lys Ser
   1               5                   10                  15
   Ala Glu Tyr
<210> 47
   <211> 19
   <212> PRT
   <213> Artificial Sequence
<220>
   <221> source
   <223> /note="Description of Artificial Sequence: Synthetic peptide"
<400> 47
   Pro Phe Phe Tyr Gly Gly Cys Leu Gly Asn Lys Asn Asn Phe Lys Thr
   1               5                   10                  15
   Ala Glu Tyr
<210> 48
   <211> 19
   <212> PRT
   <213> Artificial Sequence
<220>
   <221> source
   <223> /note="Description of Artificial Sequence: Synthetic peptide"
<400> 48
   Thr Phe Phe Tyr Gly Gly Cys Arg Gly Asn Arg Asn Asn Phe Lys Thr
   1               5                   10                  15
   Glu Glu Tyr
<210> 49
   <211> 19
   <212> PRT
   <213> Artificial Sequence
<220>
   <221> source
   <223> /note="Description of Artificial Sequence: Synthetic peptide"
<400> 49
   Thr Phe Phe Tyr Gly Gly Cys Arg Gly Lys Arg Asn Asn Phe Lys Thr
   1               5                   10                  15
   Glu Glu Asp
<210> 50
   <211> 19
   <212> PRT
   <213> Artificial Sequence
<220>
   <221> source
   <223> /note="Description of Artificial Sequence: Synthetic peptide"
<400> 50
   Pro Phe Phe Tyr Gly Gly Cys Gly Gly Asn Gly Asn Asn Phe Val Arg
   1               5                   10                  15
   Glu Lys Tyr
<210> 51
   <211> 19
   <212> PRT
   <213> Artificial Sequence
<220>
   <221> source
   <223> /note="Description of Artificial Sequence: Synthetic peptide"
<400> 51
   Ser Phe Phe Tyr Gly Gly Cys Met Gly Asn Gly Asn Asn Phe Val Arg
   1               5                   10                  15
   Glu Lys Tyr
<210> 52
   <211> 19
   <212> PRT
   <213> Artificial Sequence
<220>
   <221> source
   <223> /note="Description of Artificial Sequence: Synthetic peptide"
<400> 52
   Ser Phe Phe Tyr Gly Gly Cys Met Gly Asn Gly Asn Asn Phe Val Arg
   1               5                   10                  15
   Glu Lys Tyr
<210> 53
   <211> 19
   <212> PRT
   <213> Artificial Sequence
<220>
   <221> source
   <223> /note="Description of Artificial Sequence: Synthetic peptide"
<400> 53
   Thr Phe Phe Tyr Gly Gly Cys Leu Gly Asn Gly Asn Asn Phe Val Arg
   1               5                   10                  15
   Glu Lys Tyr
<210> 54
   <211> 19
   <212> PRT
   <213> Artificial Sequence
<220>
   <221> source
   <223> /note="Description of Artificial Sequence: Synthetic peptide"
<400> 54
   Ser Phe Phe Tyr Gly Gly Cys Leu Gly Asn Gly Asn Asn Tyr Leu Arg
   1 5 10 15
   Glu Lys Tyr
<210> 55
   <211> 19
   <212> PRT
   <213> Artificial Sequence
<220>
   <221> source
   <223> /note="Description of Artificial Sequence: Synthetic peptide"
<400> 55
   Thr Phe Phe Tyr Gly Gly Ser Leu Gly Asn Gly Asn Asn Phe Val Arg
   1 5 10 15
   Glu Lys Tyr
<210> 56
   <211> 19
   <212> PRT
   <213> Artificial Sequence
<220>
   <221> source
   <223> /note="Description of Artificial Sequence: Synthetic peptide"
<400> 56
   Thr Phe Phe Tyr Gly Gly Cys Arg Gly Asn Gly Asn Asn Phe Val Thr
   1 5 10 15
   Ala Glu Tyr
<210> 57
   <211> 19
   <212> PRT
   <213> Artificial Sequence
<220>
   <221> source
   <223> /note="Description of Artificial Sequence: Synthetic peptide"
<400> 57
   Thr Phe Phe Tyr Gly Gly Cys Leu Gly Lys Gly Asn Asn Phe Val Ser
   1 5 10 15
   Ala Glu Tyr
<210> 58
   <211> 19
   <212> PRT
   <213> Artificial Sequence
<220>
   <221> source
   <223> /note="Description of Artificial Sequence: Synthetic peptide"
<400> 58
   Thr Phe Phe Tyr Gly Gly Cys Leu Gly Asn Arg Asn Asn Phe Asp Arg
   1               5                   10                  15
   Ala Glu Tyr
<210> 59
   <211> 19
   <212> PRT
   <213> Artificial Sequence
<220>
   <221> source
   <223> /note="Description of Artificial Sequence: Synthetic peptide"
<400> 59
   Thr Phe Phe Tyr Gly Gly Cys Leu Gly Asn Arg Asn Asn Phe Leu Arg
   1               5                   10                  15
   Glu Glu Tyr
<210> 60
   <211> 19
   <212> PRT
   <213> Artificial Sequence
<220>
   <221> source
   <223> /note="Description of Artificial Sequence: Synthetic peptide"
<400> 60
   Thr Phe Phe Tyr Gly Gly Cys Leu Gly Asn Lys Asn Asn Tyr Leu Arg
   1               5                   10                  15
   Glu Glu Tyr
<210> 61
   <211> 19
   <212> PRT
   <213> Artificial Sequence
<220>
   <221> source
   <223> /note="Description of Artificial Sequence: Synthetic peptide"
<400> 61
   Pro Phe Phe Tyr Gly Gly Cys Gly Gly Asn Arg Asn Asn Tyr Leu Arg
   1               5                   10                  15
   Glu Glu Tyr
<210> 62
   <211> 16
   <212> PRT
   <213> Artificial Sequence
<220>
   <221> source
   <223> /note="Description of Artificial Sequence: Synthetic peptide"
<400> 62
   Pro Phe Phe Tyr Gly Gly Ser Gly Gly Asn Arg Asn Asn Tyr Leu Arg
   1               5                   10                  15
<210> 63
   <211> 19
   <212> PRT
   <213> Artificial Sequence
<220>
   <221> source
   <223> /note="Description of Artificial Sequence: Synthetic peptide"
<400> 63
   Met Arg Pro Asp Phe Cys Leu Glu Pro Pro Tyr Thr Gly Pro Cys Val
   1               5                   10                  15
   Ala Arg Ile
<210> 64
   <211> 21
   <212> PRT
   <213> Artificial Sequence
<220>
   <221> source
   <223> /note="Description of Artificial Sequence: Synthetic peptide"
<400> 64
   Ala Arg Ile Ile Arg Tyr Phe Tyr Asn Ala Lys Ala Gly Leu Cys Gln
   1               5                   10                  15
   Thr Phe Val Tyr Gly
               20
<210> 65
   <211> 22
   <212> PRT
   <213> Artificial Sequence
<220>
   <221> source
   <223> /note="Description of Artificial Sequence: Synthetic peptide"
<400> 65
   Tyr Gly Gly Cys Arg Ala Lys Arg Asn Asn Tyr Lys Ser Ala Glu Asp
   1               5                   10                  15
   Cys Met Arg Thr Cys Gly
               20
<210> 66
   <211> 22
   <212> PRT
   <213> Artificial Sequence
<220>
   <221> source
   <223> /note="Description of Artificial Sequence: Synthetic peptide"
<400> 66
   Pro Asp Phe Cys Leu Glu Pro Pro Tyr Thr Gly Pro Cys Val Ala Arg
   1               5                   10                  15
   Ile Ile Arg Tyr Phe Tyr
               20
<210> 67
   <211> 19
   <212> PRT
   <213> Artificial Sequence
<220>
   <221> source
   <223> /note="Description of Artificial Sequence: Synthetic peptide"
<400> 67
   Thr Phe Phe Tyr Gly Gly Cys Arg Gly Lys Arg Asn Asn Phe Lys Thr
   1               5                   10                  15
   Glu Glu Tyr
<210> 68
   <211> 19
   <212> PRT
   <213> Artificial Sequence
<220>
   <221> source
   <223> /note="Description of Artificial Sequence: Synthetic peptide"
<400> 68
   Lys Phe Phe Tyr Gly Gly Cys Arg Gly Lys Arg Asn Asn Phe Lys Thr
   1               5                   10                  15
   Glu Glu Tyr
<210> 69
   <211> 19
   <212> PRT
   <213> Artificial Sequence
<220>
   <221> source
   <223> /note="Description of Artificial Sequence: Synthetic peptide"
<400> 69
   Thr Phe Tyr Tyr Gly Gly Cys Arg Gly Lys Arg Asn Asn Tyr Lys Thr
   1               5                   10                  15
   Glu Glu Tyr
<210> 70
   <211> 19
   <212> PRT
   <213> Artificial Sequence
<220>
   <221> source
   <223> /note="Description of Artificial Sequence: Synthetic peptide"
<400> 70
   Thr Phe Phe Tyr Gly Gly Ser Arg Gly Lys Arg Asn Asn Phe Lys Thr
   1               5                   10                  15
   Glu Glu Tyr
<210> 71
   <211> 20
   <212> PRT
   <213> Artificial Sequence
<220>
   <221> source
   <223> /note="Description of Artificial Sequence: Synthetic peptide"
<400> 71
   Cys Thr Phe Phe Tyr Gly Cys Cys Arg Gly Lys Arg Asn Asn Phe Lys
   1               5                   10                  15
   Thr Glu Glu Tyr
               20
<210> 72
   <211> 20
   <212> PRT
   <213> Artificial Sequence
<220>
   <221> source
   <223> /note="Description of Artificial Sequence: Synthetic peptide"
<400> 72
   Thr Phe Phe Tyr Gly Gly Cys Arg Gly Lys Arg Asn Asn Phe Lys Thr
   1               5                   10                  15
   Glu Glu Tyr Cys
               20
<210> 73
   <211> 20
   <212> PRT
   <213> Artificial Sequence
<220>
   <221> source
   <223> /note="Description of Artificial Sequence: Synthetic peptide"
<400> 73
   Cys Thr Phe Phe Tyr Gly Ser Cys Arg Gly Lys Arg Asn Asn Phe Lys
   1               5                   10                  15
   Thr Glu Glu Tyr
               20
<210> 74
   <211> 20
   <212> PRT
   <213> Artificial Sequence
<220>
   <221> source
   <223> /note="Description of Artificial Sequence: Synthetic peptide"
<400> 74
   Thr Phe Phe Tyr Gly Gly Ser Arg Gly Lys Arg Asn Asn Phe Lys Thr
   1 5 10 15
   Glu Glu Tyr Cys
   20
<210> 75
   <211> 19
   <212> PRT
   <213> Artificial Sequence
<220>
   <221> source
   <223> /note="Description of Artificial Sequence: Synthetic peptide"
<400> 75
   Pro Phe Phe Tyr Gly Gly Cys Arg Gly Lys Arg Asn Asn Phe Lys Thr
   1 5 10 15
   Glu Glu Tyr
<210> 76
   <211> 19
   <212> PRT
   <213> Artificial Sequence
<220>
   <221> source
   <223> /note="Description of Artificial Sequence: Synthetic peptide"
<400> 76
   Thr Phe Phe Tyr Gly Gly Cys Arg Gly Lys Arg Asn Asn Phe Lys Thr
   1 5 10 15
   Lys Glu Tyr
<210> 77
   <211> 19
   <212> PRT
   <213> Artificial Sequence
<220>
   <221> source
   <223> /note="Description of Artificial Sequence: Synthetic peptide"
<400> 77
   Thr Phe Phe Tyr Gly Gly Lys Arg Gly Lys Arg Asn Asn Phe Lys Thr
   1 5 10 15
   Glu Glu Tyr
<210> 78
   <211> 19
   <212> PRT
   <213> Artificial Sequence
<220>
   <221> source
   <223> /note="Description of Artificial Sequence: Synthetic peptide"
<400> 78
   Thr Phe Phe Tyr Gly Gly Cys Arg Gly Lys Arg Asn Asn Phe Lys Thr
   1               5                   10                  15
   Lys Arg Tyr
<210> 79
   <211> 19
   <212> PRT
   <213> Artificial Sequence
<220>
   <221> source
   <223> /note="Description of Artificial Sequence: Synthetic peptide"
<400> 79
   Thr Phe Phe Tyr Gly Gly Lys Arg Gly Lys Arg Asn Asn Phe Lys Thr
   1               5                   10                  15
   Ala Glu Tyr
<210> 80
   <211> 19
   <212> PRT
   <213> Artificial Sequence
<220>
   <221> source
   <223> /note="Description of Artificial Sequence: Synthetic peptide"
<400> 80
   Thr Phe Phe Tyr Gly Gly Lys Arg Gly Lys Arg Asn Asn Phe Lys Thr
   1               5                   10                  15
   Ala Gly Tyr
<210> 81
   <211> 19
   <212> PRT
   <213> Artificial Sequence
<220>
   <221> source
   <223> /note="Description of Artificial Sequence: Synthetic peptide"
<400> 81
   Thr Phe Phe Tyr Gly Gly Lys Arg Gly Lys Arg Asn Asn Phe Lys Arg
   1 5 10 15
   Glu Lys Tyr
<210> 82
   <211> 19
   <212> PRT
   <213> Artificial Sequence
<220>
   <221> source
   <223> /note="Description of Artificial Sequence: Synthetic peptide"
<400> 82
   Thr Phe Phe Tyr Gly Gly Lys Arg Gly Lys Arg Asn Asn Phe Lys Arg
   1 5 10 15
   Ala Lys Tyr
<210> 83
   <211> 19
   <212> PRT
   <213> Artificial Sequence
<220>
   <221> source
   <223> /note="Description of Artificial Sequence: Synthetic peptide"
<400> 83
   Thr Phe Phe Tyr Gly Gly Cys Leu Gly Asn Arg Asn Asn Phe Lys Thr
   1 5 10 15
   Glu Glu Tyr
<210> 84
   <211> 19
   <212> PRT
   <213> Artificial Sequence
<220>
   <221> source
   <223> /note="Description of Artificial Sequence: Synthetic peptide"
<400> 84
   Thr Phe Phe Tyr Gly Cys Gly Arg Gly Lys Arg Asn Asn Phe Lys Thr
   1 5 10 15
   Glu Glu Tyr
<210> 85
   <211> 19
   <212> PRT
   <213> Artificial Sequence
<220>
   <221> source
   <223> /note="Description of Artificial Sequence: Synthetic peptide"
<400> 85
   Thr Phe Phe Tyr Gly Gly Arg Cys Gly Lys Arg Asn Asn Phe Lys Thr
   1 5 10 15
   Glu Glu Tyr
<210> 86
   <211> 19
   <212> PRT
   <213> Artificial Sequence
<220>
   <221> source
   <223> /note="Description of Artificial Sequence: Synthetic peptide"
<400> 86
   Thr Phe Phe Tyr Gly Gly Cys Leu Gly Asn Gly Asn Asn Phe Asp Thr
   1 5 10 15
   Glu Glu Glu
<210> 87
   <211> 19
   <212> PRT
   <213> Artificial Sequence
<220>
   <221> source
   <223> /note="Description of Artificial Sequence: Synthetic peptide"
<400> 87
   Thr Phe Gln Tyr Gly Gly Cys Arg Gly Lys Arg Asn Asn Phe Lys Thr
   1 5 10 15
   Glu Glu Tyr
<210> 88
   <211> 19
   <212> PRT
   <213> Artificial Sequence
<220>
   <221> source
   <223> /note="Description of Artificial Sequence: Synthetic peptide"
<400> 88
   Tyr Asn Lys Glu Phe Gly Thr Phe Asn Thr Lys Gly Cys Glu Arg Gly
   1               5                   10                  15
   Tyr Arg Phe
<210> 89
   <211> 19
   <212> PRT
   <213> Artificial Sequence
<220>
   <221> source
   <223> /note="Description of Artificial Sequence: Synthetic peptide"
<400> 89
   Arg Phe Lys Tyr Gly Gly Cys Leu Gly Asn Met Asn Asn Phe Glu Thr
   1               5                   10                  15
   Leu Glu Glu
<210> 90
   <211> 19
   <212> PRT
   <213> Artificial Sequence
<220>
   <221> source
   <223> /note="Description of Artificial Sequence: Synthetic peptide"
<400> 90
   Arg Phe Lys Tyr Gly Gly Cys Leu Gly Asn Lys Asn Asn Phe Leu Arg
   1               5                   10                  15
   Leu Lys Tyr
<210> 91
   <211> 19
   <212> PRT
   <213> Artificial Sequence
<220>
   <221> source
   <223> /note="Description of Artificial Sequence: Synthetic peptide"
<400> 91
   Arg Phe Lys Tyr Gly Gly Cys Leu Gly Asn Lys Asn Asn Tyr Leu Arg
   1               5                   10                  15
   Leu Lys Tyr
<210> 92
   <211> 22
   <212> PRT
   <213> Artificial Sequence
<220>
   <221> source
   <223> /note="Description of Artificial Sequence: Synthetic peptide"
<400> 92
   Lys Thr Lys Arg Lys Arg Lys Lys Gln Arg Val Lys Ile Ala Tyr Glu
   1               5                   10                  15
   Glu Ile Phe Lys Asn Tyr
               20
<210> 93
   <211> 15
   <212> PRT
   <213> Artificial Sequence
<220>
   <221> source
   <223> /note="Description of Artificial Sequence: Synthetic peptide"
<400> 93
   Lys Thr Lys Arg Lys Arg Lys Lys Gln Arg Val Lys Ile Ala Tyr
   1               5                   10                  15
<210> 94
   <211> 17
   <212> PRT
   <213> Artificial Sequence
<220>
   <221> source
   <223> /note="Description of Artificial Sequence: Synthetic peptide"
<400> 94
   Arg Gly Gly Arg Leu Ser Tyr Ser Arg Arg Phe Ser Thr Ser Thr Gly
   1               5                   10                  15
   Arg
<210> 95
   <211> 10
   <212> PRT
   <213> Artificial Sequence
<220>
   <221> source
   <223> /note="Description of Artificial Sequence: Synthetic peptide"
<400> 95
   Arg Arg Leu Ser Tyr Ser Arg Arg Arg Phe
   1               5                   10
<210> 96
   <211> 16
   <212> PRT
   <213> Artificial Sequence
<220>
   <221> source
   <223> /note="Description of Artificial Sequence: Synthetic peptide"
<400> 96
   Arg Gln Ile Lys Ile Trp Phe Gln Asn Arg Arg Met Lys Trp Lys Lys
   1               5                   10                  15
<210> 97
   <211> 19
   <212> PRT
   <213> Artificial Sequence
<220>
   <221> source
   <223> /note="Description of Artificial Sequence: Synthetic peptide"
<400> 97
   Thr Phe Phe Tyr Gly Gly Ser Arg Gly Lys Arg Asn Asn Phe Lys Thr
   1               5                   10                  15
   Glu Glu Tyr
<210> 98
   <211> 58
   <212> PRT
   <213> Artificial Sequence
<220>
   <221> source
   <223> /note="Description of Artificial Sequence: Synthetic polypeptide"
<400> 98
   Arg Pro Asp Phe Cys Leu Glu Pro Pro Tyr Thr Gly Pro Cys Lys Ala
   1 5 10 15
   Arg Ile Ile Arg Tyr Tyr Phe Tyr Asn Ala Lys Ala Gly Leu Cys Gln
   20 25 30
   Thr Phe Val Tyr Gly Gly Cys Arg Ala Lys Arg Asn Asn Phe Lys Ser
   35 40 45
   Ala Glu Asp Cys Met Thr Cys Gly Gly Ala
   50 55
<210> 99
   <211> 19
   <212> PRT
   <213> Artificial Sequence
<220>
   <221> source
   <223> /note="Description of Artificial Sequence: Synthetic peptide"
<400> 99
   Thr Phe Phe Tyr Gly Gly Cys Arg Gly Lys Arg Asn Asn Phe Lys Thr
   1 5 10 15
   Lys Glu Tyr
<210> 100
   <211> 19
   <212> PRT
   <213> Artificial Sequence
<220>
   <221> source
   <223> /note="Description of Artificial Sequence: Synthetic peptide"
<400> 100
   Arg Phe Lys Tyr Gly Gly Cys Leu Gly Asn Lys Asn Asn Tyr Leu Arg
   1 5 10 15
   Leu Lys Tyr
<210> 101
   <211> 19
   <212> PRT
   <213> Artificial Sequence
<220>
   <221> source
   <223> /note="Description of Artificial Sequence: Synthetic peptide"
<400> 101
   Thr Phe Phe Tyr Gly Gly Cys Arg Ala Lys Arg Asn Asn Phe Lys Arg
   1               5                   10                  15
   Ala Lys Tyr
<210> 102
   <211> 35
   <212> PRT
   <213> Artificial Sequence
<220>
   <221> source
   <223> /note="Description of Artificial Sequence: Synthetic polypeptide"
<400> 102
   Asn Ala Lys Ala Gly Leu Cys Gln Thr Phe Val Tyr Gly Gly Cys Leu
   1               5                   10                  15
   Ala Lys Arg Asn Asn Phe Glu Ser Ala Glu Asp Cys Met Arg Thr Cys
               20                  25                  30
   Gly Gly Ala
           35
<210> 103
   <211> 24
   <212> PRT
   <213> Artificial Sequence
<220>
   <221> source
   <223> /note="Description of Artificial Sequence: Synthetic peptide"
<400> 103
   Tyr Gly Gly Cys Arg Ala Lys Arg Asn Asn Phe Lys Ser Ala Glu Asp
   1               5                   10                  15
   Cys Met Arg Thr Cys Gly Gly Ala
               20
<210> 104
   <211> 22
   <212> PRT
   <213> Artificial Sequence
<220>
   <221> source
   <223> /note="Description of Artificial Sequence: Synthetic peptide"
<400> 104
   Gly Leu Cys Gln Thr Phe Val Tyr Gly Gly Cys Arg Ala Lys Arg Asn
   1               5                   10                  15
   Asn Phe Lys Ser Ala Glu
               20
<210> 105
   <211> 20
   <212> PRT
   <213> Artificial Sequence
<220>
   <221> source
   <223> /note="Description of Artificial Sequence: Synthetic peptide"
<400> 105
   Leu Cys Gln Thr Phe Val Tyr Gly Gly Cys Glu Ala Lys Arg Asn Asn
   1               5                   10                  15
   Phe Lys Ser Ala
               20
<210> 106
   <211> 6
   <212> PRT
   <213> Artificial Sequence
<220>
   <221> source
   <223> /note="Description of Artificial Sequence: Synthetic peptide"
<220>
   <221> VARIANT
   <222> (3)..(3)
   <223> /replace="Gln"
<220>
   <221> VARIANT
   <222> (4)..(4)
   <223> /replace="Gln"
<220>
   <221> VARIANT
   <222> (5)..(5)
   <223> /replace="Tyr" or "Trp"
<220>
   <221> misc_feature
   <222> (1)..(6)
   <223> /note="Variant residues given in the sequence have no preference with respect to those in the annotations for variant positions"
<400> 106
   Lys Arg Asn Asn Phe Lys
   1 5
<210> 107
   <211> 19
   <212> PRT
   <213> Artificial Sequence
<220>
   <221> source
   <223> /note="Description of Artificial Sequence: Synthetic peptide"
<400> 107
   Thr Phe Phe Tyr Gly Gly Ser Arg Gly Lys Arg Asn Asn Phe Lys Thr
   1 5 10 15
   Glu Glu Tyr
<210> 108
   <211> 19
   <212> PRT
   <213> Artificial Sequence
<220>
   <221> source
   <223> /note="Description of Artificial Sequence: Synthetic peptide"
<400> 108
   Arg Phe Phe Tyr Gly Gly Ser Arg Gly Lys Arg Asn Asn Phe Lys Thr
   1 5 10 15
   Glu Glu Tyr
<210> 109
   <211> 19
   <212> PRT
   <213> Artificial Sequence
<220>
   <221> source
   <223> /note="Description of Artificial Sequence: Synthetic peptide"
<400> 109
   Arg Phe Phe Tyr Gly Gly Ser Arg Gly Lys Arg Asn Asn Phe Lys Thr
   1 5 10 15
   Glu Glu Tyr
<210> 110
   <211> 19
   <212> PRT
   <213> Artificial Sequence
<220>
   <221> source
   <223> /note="Description of Artificial Sequence: Synthetic peptide"
<400> 110
   Arg Phe Phe Tyr Gly Gly Ser Arg Gly Lys Arg Asn Asn Phe Arg Thr
   1               5                   10                  15
   Glu Glu Tyr
<210> 111
   <211> 19
   <212> PRT
   <213> Artificial Sequence
<220>
   <221> source
   <223> /note="Description of Artificial Sequence: Synthetic peptide"
<400> 111
   Thr Phe Phe Tyr Gly Gly Ser Arg Gly Lys Arg Asn Asn Phe Arg Thr
   1               5                   10                  15
   Glu Glu Tyr
<210> 112
   <211> 19
   <212> PRT
   <213> Artificial Sequence
<220>
   <221> source
   <223> /note="Description of Artificial Sequence: Synthetic peptide"
<400> 112
   Thr Phe Phe Tyr Gly Gly Ser Arg Gly Arg Arg Asn Asn Phe Arg Thr
   1               5                   10                  15
   Glu Glu Tyr
<210> 113
   <211> 20
   <212> PRT
   <213> Artificial Sequence
<220>
   <221> source
   <223> /note="Description of Artificial Sequence: Synthetic peptide"
<400> 113
   Cys Thr Phe Phe Tyr Gly Gly Ser Arg Gly Lys Arg Asn Asn Phe Lys
   1 5 10 15
   Thr Glu Glu Tyr
   20
<210> 114
   <211> 20
   <212> PRT
   <213> Artificial Sequence
<220>
   <221> source
   <223> /note="Description of Artificial Sequence: Synthetic peptide"
<400> 114
   Thr Phe Phe Tyr Gly Gly Ser Arg Gly Lys Arg Asn Asn Phe Lys Thr
   1 5 10 15
   Glu Glu Tyr Cys
   20
<210> 115
   <211> 20
   <212> PRT
   <213> Artificial Sequence
<220>
   <221> source
   <223> /note="Description of Artificial Sequence: Synthetic peptide"
<400> 115
   Cys Thr Phe Phe Tyr Gly Gly Ser Arg Gly Arg Arg Asn Asn Phe Arg
   1 5 10 15
   Thr Glu Glu Tyr
   20
<210> 116
   <211> 20
   <212> PRT
   <213> Artificial Sequence
<220>
   <221> source
   <223> /note="Description of Artificial Sequence: Synthetic peptide"
<400> 116
   Thr Phe Phe Tyr Gly Gly Ser Arg Gly Arg Arg Asn Asn Phe Arg Thr
   1               5                   10                  15
   Glu Glu Tyr Cys
               20
<210> 117
   <211> 19
   <212> PRT
   <213> Artificial Sequence
<220>
   <221> source
   <223> /note="Description of Artificial Sequence: Synthetic peptide"
<220>
   <221> VARIANT
   <222> (7)..(7)
   <223> /replace="Ser"
<220>
   <221> misc_feature
   <222> (1)..(19)
   <223> /note="Variant residues given in the sequence have no preference with respect to those in the annotations for variant positions"
<400> 117
   Thr Phe Phe Tyr Gly Gly Cys Arg Gly Lys Arg Asn Asn Phe Lys Thr
   1               5                   10                  15
   Glu Glu Tyr
<210> 118
   <211> 18
   <212> PRT
   <213> Artificial Sequence
<220>
   <221> source
   <223> /note="Description of Artificial Sequence: Synthetic peptide"
<220>
   <221> VARIANT
   <222> (5)..(5)
   <223> /replace="Ser"
<220>
   <221> misc_feature
   <222> (1)..(18)
   <223> /note="Variant residues given in the sequence have no preference with respect to those in the annotations for variant positions"
<400> 118
   Phe Tyr Gly Gly Cys Arg Gly Lys Arg Asn Asn Phe Lys Thr Glu Glu
   1 5 10 15
   Tyr Cys
<210> 119
   <211> 16
   <212> PRT
   <213> Artificial Sequence
<220>
   <221> source
   <223> /note="Description of Artificial Sequence: Synthetic peptide"
<400> 119
   Gly Gly Ser Arg Gly Lys Arg Asn Asn Phe Lys Thr Glu Glu Tyr Cys
   1 5 10 15
<210> 120
   <211> 14
   <212> PRT
   <213> Artificial Sequence
<220>
   <221> source
   <223> /note="Description of Artificial Sequence: Synthetic peptide"
<400> 120
   Ser Arg Gly Lys Arg Asn Asn Phe Lys Thr Glu Glu Tyr Cys
   1 5 10
<210> 121
   <211> 12
   <212> PRT
   <213> Artificial Sequence
<220>
   <221> source
   <223> /note="Description of Artificial Sequence: Synthetic peptide"
<400> 121
   Gly Lys Arg Asn Asn Phe Lys Thr Glu Glu Tyr Cys
   1 5 10
<210> 122
   <211> 8
   <212> PRT
   <213> Artificial Sequence
<220>
   <221> source
   <223> /note="Description of Artificial Sequence: Synthetic peptide"
<400> 122
   Lys Arg Asn Asn Phe Lys Tyr Cys
   1 5
<210> 123
   <211> 6
   <212> PRT
   <213> Artificial Sequence
<220>
   <221> source
   <223> /note="Description of Artificial Sequence: Synthetic peptide"
<400> 123
   Lys Arg Asn Asn Phe Lys
   1 5
<210> 124
   <211> 214
   <212> PRT
   <213> Artificial Sequence
<220>
   <221> source
   <223> /note="Description of Artificial Sequence: Synthetic polypeptide"
<400> 124
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Asn Thr Ala
   20 25 30
   Val Ala Trp Tyr Gln Gln Lys Pro Gly Lys Ala Pro Lys Leu Leu Ile
   35 40 45
   Tyr Ser Ala Ser Phe Leu Tyr Ser Gly Val Pro Ser Arg Phe Ser Gly
       50                  55                  60
   Ser Arg Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 125
   <211> 451
   <212> PRT
   <213> Artificial Sequence
<220>
   <221> source
   <223> /note="Description of Artificial Sequence: Synthetic polypeptide"
<400> 125
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
   35 40 45
   Ala Arg Ile Tyr Pro Thr Asn Gly Tyr Thr Arg Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ser Arg Trp Gly Gly Asp Gly Phe Tyr Ala Met Asp Tyr Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Lys Val Glu Pro Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Ser Val Leu Thr Val Leu His Gln Asp Trp Leu Asn Gly
   305 310 315 320
   Lys Glu Tyr Lys Cys Lys Val Ser Asn Lys Ala Leu Pro Ala Pro Ile
   325 330 335
   Glu Lys Thr Ile Ser Lys Ala Lys Gly Gln Pro Arg Glu Pro Gln Val
   340 345 350
   Tyr Thr Leu Pro Pro Ser Arg Asp Glu Leu Thr Lys Asn Gln Val Ser
   355 360 365
   Leu Thr Cys Leu Val Lys Gly Phe Tyr Pro Ser Asp Ile Ala Val Glu
   370 375 380
   Trp Glu Ser Asn Gly Gln Pro Glu Asn Asn Tyr Lys Thr Thr Pro Pro
   385 390 395 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 126
   <211> 58
   <212> PRT
   <213> Artificial Sequence
<220>
   <221> source
   <223> /note="Description of Artificial Sequence: Synthetic polypeptide"
<400> 126
<210> 127
   <211> 58
   <212> PRT
   <213> Artificial Sequence
<220>
   <221> source
   <223> /note="Description of Artificial Sequence: Synthetic polypeptide"
<400> 127
<210> 128
   <211> 58
   <212> PRT
   <213> Homo sapiens
<400> 128
   Arg Pro Asp Phe Cys Leu Glu Pro Pro Tyr Thr Gly Pro Cys Lys Ala
   1               5                   10                  15
   Arg Ile Ile Arg Tyr Tyr Phe Tyr Asn Ala Lys Ala Gly Leu Cys Gln
               20                  25                  30
   Thr Phe Val Tyr Gly Gly Cys Arg Ala Lys Arg Asn Asn Phe Lys Ser
           35                  40                  45
   Ala Glu Asp Cys Met Thr Cys Gly Gly Ala
       50                  55
<210> 129
   <211> 19
   <212> PRT
   <213> Homo sapiens
<400> 129
   Thr Phe Phe Tyr Gly Gly Ser Arg Gly Lys Arg Asn Asn Phe Lys Thr
   1 5 10 15
   Glu Glu Tyr